# EUROPEAN PATENT SPECIFICATION

(11) **EP 3 645 718 B1**
(45) Date of publication and mention of the grant of the patent: **17.12.2025**
(21) Application number: 18822633.6
(22) Date of filing: 29.06.2018
(51) Int. Cl.: C12N 15/10, C12Q 1/68, C12Q 1/6806, C12Q 1/6844, C12Q 1/6883, C40B 20/04, C12Q 1/6827

(54) **METHODS AND SYSTEMS FOR EVALUATING DNA METHYLATION IN CELL-FREE DNA**
VERFAHREN UND SYSTEME ZUR BEURTEILUNG DER DNA-METHYLIERUNG IN ZELLFREIER DNA
PROCÉDÉS ET SYSTÈMES D'ÉVALUATION DE LA MÉTHYLATION DE L'ADN DANS L'ADN ACELLULAIRE

(30) Priority: 30.06.2017 US 201762527236 P
(43) Date of publication of application: 06.05.2020
(73) Proprietor: The Regents of the University of California, Oakland, CA 94607-5200 (US)
(72) Inventor: ZHOU, Xianghong, Los Angeles CA 90095 (US); HE, Shanshan, Los Angeles CA 90095 (US); SAME, Mary Louisa, Los Angeles CA 90095 (US); ZHOU, Yonggang, Los Angeles CA 90095 (US); NI, Xiaohui, Los Angeles CA 90095 (US); ZENG, Weihua, Los Angeles CA 90095 (US)
(74) Representative: J A Kemp LLP
(86) International application number: PCT/US2018/040239
(87) International publication number: WO 2019/006269

(56) References cited:
- WO-A1-2017/083562
- WO-A1-2017/162754
- WO-A1-2017/212428
- US-A1- 2013 085 681
- US-A1- 2015 284 769
- DE KOKER A. ET AL: "A versatile method for circulating cell-free DNA methylome profiling by reduced representation bisulfite sequencing", BIORXIV, 11 June 2019 (2019-06-11), XP055830009, Retrieved from the Internet <URL:https://www.biorxiv.org/content/10.1101/663195v2> [retrieved on 20210804], DOI: 10.1101/663195
- LU WEN ET AL: "Genome-scale detection of hypermethylated CpG islands in circulating cell-free DNA of hepatocellular carcinoma patients", CELL RESEARCH, vol. 25, no. 11, 30 October 2015 (2015-10-30), Singapore, pages 1250 - 1264, XP055465354, ISSN: 1001-0602, DOI: 10.1038/cr.2015.126
- VEILLARD ANNE-CL�MENCE ET AL: "Diagenode� Premium RRBS technology: cost-effective DNA methylation mapping with superior coverage", NATURE METHODS, vol. 13, no. 2, 1 February 2016 (2016-02-01), New York, pages i - ii, XP093026593, ISSN: 1548-7091, DOI: 10.1038/nmeth.f.391
- GU ET AL.: "Genome-scale DNA methylation mapping of clinical samples at single-nucleotide resolution", NATURE METHODS, vol. 7, no. 2, 10 January 2010 (2010-01-10), pages 133 - 136, XP002733779
- PLONGTHONGKUM ET AL.: "Advances in the Profiling of DNA Modifications: Cytosine Methylation and Beyond", NATURE REVIEWS GENETICS, vol. 15, no. 10, 27 August 2014 (2014-08-27), pages 647 - 661, XP055555523

## Description

### TECHNICAL FIELD

The field of the disclosure includes at least cell biology, molecular biology, DNA analysis, library preparation, diagnostics and/or medicine.

### BACKGROUND

Cancer cells often display aberrant DNA methylation patterns. Hypermethylated and/or hypomethylated tumor DNA fragments can be released into the bloodstream *via* cell apoptosis or necrosis, where they may become part of the circulating cell-free DNA (cfDNA) in bodily fluids, such as plasma or urine. Thus, cfDNA methylation profiling is a promising strategy for cancer screening. Whole-genome bisulfite sequencing provides a comprehensive view of the DNA methylome, but it can be expensive to deep sequence the entire genome. Reduced Representation Bisulfite Sequencing (RRBS) is a cost effective technique for the methylation profiling of genome regions that have high CpG content (the majority of DNA methylation occurs at CpG sites). In RRBS, genomic DNA is first digested with a restriction endonuclease (usually MspI) and then size-selected to enrich for CpG-dense regions. These regions compose only ~ 3% of the genome but provides comprehensive DNA methylation information about the genome. However, cfDNA is already fragmented in nature, exhibiting a characteristic peak around 166 bp. If the typical RRBS procedure is followed to select fragments between 40-220 bp, it would be very similar to select the whole population of cfDNA. Hence, while almost every fragment generated from genomic DNA and present in typical RRBS library has been cut twice by MspI, this is not true for cfDNA. As such performing typical RRBS on cfDNA will lack CpG enrichment for cfDNA that would otherwise be beneficial to perform methylation profiling for the purpose of clinical diagnostic applications, for example.

WO 2017/162754 A1 describes methods and kits for preparing a collection of degraded DNA fragments isolated from a biofluid sample of an individual. Lu Wen ET AL (2015-10-30) and Veillard Anne-Clémence ET AL (2016-02-01) relate to reduced representation bisulfite sequencing (RRBS) that enables genome-scale DNA methylation analysis.

The present disclosure concerns improvements in the art for applying RRBS to cfDNA, including to facilitate preparation of libraries from blood-borne or plasma-borne or urine-borne (or a combination thereof) cfDNA for methylation profiling.

### SUMMARY OF THE INVENTION

The invention provides a method for enriching a plurality of deoxyribonucleic acid (DNA) fragments from a plurality of cell-free DNA (cfDNA) molecules of a subject, comprising: (a) modifying one or both ends of each of at least a portion of said plurality of cell-free DNA molecules or derivatives thereof to provide a plurality of modified cell-free DNA molecules having ends that are incapable of coupling with adapters; (b) subjecting said plurality of modified cell-free DNA molecules to restriction enzyme digestion to fragment each of at least a subset of said modified cell-free DNA molecules at one or more CpG sites, thereby producing a plurality of restriction enzyme-digested DNA fragments that contain one or more CpG sites and having ends that are capable of coupling with adapters; and (c) coupling said adapters to ends of said plurality of restriction enzyme-digested DNA fragments to provide a plurality of tagged DNA fragments having methylated nucleic acid bases that are distinguishable from unmethylated nucleic acid bases, wherein said modifying comprises: (i) subjecting a 3' end of each of said at least said portion of said plurality of cfDNA molecules to conditions sufficient to modify said 3' end with a dideoxynucleotide (ddNTP) moiety or a functional analog thereof, and/or (ii) incorporation of one or more blocker oligonucleotides at said one or both ends of each of at least a portion of said plurality of cfDNA molecules.

### FURTHER DISCLOSURE

The invention is defined in the independent claims. Further aspects and preferred embodiments are defined in the dependent claims. Any aspects, embodiments and examples of the present disclosure which do not fall under the scope of the appended claims do not form part of the invention and are merely provided for illustrative purposes.

Cancer cells can often display aberrant DNA methylation patterns, such as hypermethylation of the promoter regions of tumor suppressor genes and pervasive hypormethylation of intergenic regions. Therefore, a patient's DNA methylation profile can be a target for cancer evaluation in clinical practice. Hyper-methylated and/or hypo-methylated tumor DNA fragments can be released into the bloodstream via processes such as cell apoptosis or necrosis, where these circulation tumor DNA (ctDNA) become part of the circulating cell-free DNA (cfDNA) in plasma. The non-invasive nature of cfDNA methylation profiling may be an effective strategy for screening of one or more diseases or disorders, including at least general cancer screening. The present disclosure describes methods for enriching cfDNA for regions that are informative for methylation profiling, such as CpG islands, so that the nucleic acid analyzed for methylation profiling is more effective than in the absence of such enrichment measures. Described herein, the individual has or is suspected of having or is at risk of having cancer, and the analysis of the cfDNA molecules assists in determining whether the individual has or is suspected of having or is at risk of having cancer. The cfDNA may be double-stranded, single-stranded, or a mixture thereof.

The disclosure concerns methods, systems, and compositions related to preparation of molecules for analysis of methylation quantities and/or locations in the molecules. The molecules described herein comprise cfDNA, and the cfDNA described herein is from an individual (such as blood or plasma or urine (or a combination thereof) samples from the individual). The present disclosure describes methods and systems for evaluating DNA methylation in cfDNA molecules, such as in CpG-rich regions of the cfDNA molecules. Such methods and systems may enrich cell-free DNA molecules for CpG-rich regions and described herein the methods advantageously allow methylation profiling, such as toward clinical diagnostic applications. The present disclosure relates to improved methods, systems, and compositions for enriching cell-free DNA molecules for CpG-rich regions, including facilitating preparation of libraries from cfDNA for methylation profiling. The source of the cfDNA may be blood-borne or plasma-borne or urine-borne (or a combination thereof), for example.

For disclosure related to cancer, detection and characterization of cfDNA in suitable samples can be an effective method for non-invasive cancer screening, including identifying the tumor tissue-of-origin. Liquid biopsy (which may also be referred to as fluid biopsy or fluid phase biopsy), e.g., blood draw, unlike traditional tissue biopsy, is useful to diagnose a variety of different malignancies and may be utilized in methods encompassed in the disclosure.

The present disclosure concerns enriching CpG islands in cfDNA such that methylation profiling is particularly effective for providing methylation information, including methods of evaluating DNA methylation in CpG-rich regions of cfDNA.

The present methods are not utilized for genomic DNA but instead are utilized for cfDNA. Such a distinction distinguishing methods that are suitable for enriching CpG islands for genomic DNA from those that would not work for enriching CpG islands for cfDNA. The present disclosure adapts methods for methylation analysis of genomic DNA to apply to methods for methylation analysis of cfDNA having unique differences from genomic DNA.

Described herein, the present methods are utilized for highly degraded genomic DNA such as ancient DNA or DNA from formalin-fixed paraffin-embedded tissue specimens.

Described herein are improvements and/or adaptations of reduced representation bisulfite sequencing (RRBS), which is an efficient and high-throughput technique used to analyze the genome-wide methylation profiles on a single nucleotide level. The technique combines restriction enzymes and bisulfite sequencing in order to enrich for the areas of the genome that have a high CpG content, and in at least some cases the method reduces the amount of nucleotides needed to be sequenced. The present disclosure, however, relates to an adaptation of RRBS for cfDNA or highly degraded genomic DNA because standard RRBS is not able to be utilized for cfDNA or highly degraded genomic DNA.

This disclosure, the methods of which may be referred to as cfRRBS, includes an RRBS-analog approach for cost-effective methylation profiling of cfDNA or highly degraded genomic DNA. Unlike typical RRBS, the cfRRBS procedures disclosed herein include dideoxynucleotides (ddNTP) labeling of cfDNA or highly degraded genomic DNA, followed by MspI digestion and library construction. The library described herein is then subjected to size selection of 150 ~ 400 bp. Described herein, DNA fragments comprising none or only one MspI recognizable sequence are discarded and only fragments comprising two or more MspI recognizable sequence are enriched. Described herein, this ensures that each molecule comprises at least one CpG site, which leads to cost-effective sequencing that facilitates broad clinical application of diagnosis tools.

Described herein is a method for processing or analyzing a plurality of cell-free deoxyribonucleic (DNA) molecules of a subject, comprising: (a) subjecting said plurality of cell-free DNA (cfDNA) molecules having ends that are (i) incapable of coupling with adapters or (ii) configured for separation from a remainder of said plurality of cfDNA, to conditions sufficient to fragment at least a subset of said cell-free DNA molecules at one or more CpG sites, to provide a plurality of DNA fragments; coupling said adapters to ends of said plurality of DNA fragments to provide a plurality of tagged DNA fragments having methylated nucleic acid bases that are distinguishable from unmethylated nucleic acid bases; optionally (b) subjecting said plurality of tagged DNA fragments or derivatives thereof to nucleic acid sequencing to yield a plurality of sequence reads; and optionally (c) processing said plurality of sequence reads to (i) identify sequences from said adapters at both ends of said plurality of sequence reads, and (ii) upon identifying said sequences, identifying cell-free DNA molecules from said plurality of cell-free DNA molecules as having one or more CpG sites.

As described herein, at least a subset of said plurality of DNA fragments have methylated nucleic acid bases. As described herein, identifying cell-free DNA molecules as having one or more CpG sites comprises identifying cell-free DNA molecules as having two or three or four or more CpG sites. As described herein, the method further comprises, prior to or after coupling said adapters to ends of said plurality of DNA fragments, separating fragments of said cfDNA molecules having said ends from said plurality of DNA fragments. As described herein, said fragments are coupled to magnetic beads, and wherein said fragments are separated using magnetic separation. As described herein, the method further comprises, prior to or after coupling said adapters to ends of said plurality of DNA fragments, subjecting said plurality of cfDNA molecules, said plurality of DNA fragments, or derivatives thereof to conditions sufficient to permit said methylated nucleic acid bases to be distinguished from said unmethylated nucleic acid bases. As described herein, subjecting said plurality of cfDNA molecules, said plurality of DNA fragments, or derivatives thereof to said conditions comprises performing bisulfite conversion on said plurality of DNA fragments.

As described herein, the method further comprises subjecting said plurality of tagged DNA fragments or derivatives thereof to conditions sufficient to permit said methylated bases to be distinguished from said unmethylated nucleic acid bases. As described herein, subjecting said plurality of tagged DNA fragments or derivatives thereof to said conditions comprises performing bisulfite conversion on said plurality of tagged DNA fragments. As described herein, said conditions in (a) are sufficient to fragment said at least said subset of said modified cfDNA molecules at a plurality of CpG sites.

As described herein, the step of (a) further comprises performing restriction enzyme digestion on said plurality of cfDNA molecules to fragment said at least said subset of said plurality of cfDNA molecules at said one or more CpG sites. As described herein, said restriction enzyme digestion is performed using one or more restriction enzymes that enrich DNA fragments from said plurality of cfDNA molecules having CpG sites. As described herein, said one or more restriction enzymes comprise MspI, HpaII, and/or TaqI. Described herein, MspI is utilized only, whereas HpaII it utilized with MspI or TaqI is utilized with MspI, or both HpaII and TaqI are utilized with MspI. Also described herein, HpaII is utilized only, and in other cases TaqI is utilized only. Described herein, MspI is not employed for digestion. In cases wherein multiple enzymes are utilized in the method, they may be exposed to the plurality of cfDNA molecules substantially simultaneously or in succession of any order.

As described herein, the adapters are particularly configured, including to be most effective dependent upon the nature of the sample, the purpose of the method, the intended application for the method, and so on. As described herein, each of said adapters comprises at least one functional sequence (that may be of any suitable size or sequence) that is configured to couple to a flow cell of a nucleic acid sequencer. As described herein, coupling said adapters in (b) comprises ligating said adapters to said ends of said plurality of DNA fragments. As described herein, the method further comprises, prior to said ligation, performing end repair or nucleic acid base tailing of the plurality of DNA fragments. As described herein, the method further comprises prior to said ligation, performing end repair and nucleic acid base tailing of the plurality of DNA fragments.

As described herein, said adapters are configured to be coupled to a nucleic acid molecule to provide a library for sequencing. As described herein, said adapters are configured to be ligated to said nucleic acid molecule. As described herein, said adapters comprise at least one stem-loop region. As described herein, the method further comprises coupling said adapters to said nucleic acid molecule, and linearizing said stem-loop region of said adapters coupled to said nucleic acid molecule. As described herein, said linearizing is performed using an endonuclease, a uracil glycosylase or a functional analog thereof, or a combination thereof. As described herein, said endonuclease is endonuclease VIII or a functional analog thereof. As described herein, said uracil glycosylase is a uracil deoxyribonucleic nucleic acid (DNA) glycosylase.

As described herein, said adapters are Y shaped. As described herein, said adapters are blunt ended. As described herein, said adapters comprise a known sequence. As described herein, said adapters comprise a unique sequence that allows unique molecular identification of said plurality of tagged DNA fragments or derivatives thereof.

As described herein, said nucleic acid bases of said adapters are unmethylated. As described herein, said nucleic acid bases of said adapters are methylated. As described herein, the method further comprises subjecting said plurality of DNA fragments or said plurality of tagged DNA fragments to amplification. As described herein, said amplification comprises polymerase chain reaction (PCR).

As described herein, the method further comprises performing size selection of said plurality of DNA fragments or said plurality of tagged DNA fragments to provide a size-selected plurality of DNA fragments. As described herein, said size-selected plurality of tagged DNA fragments have lengths from at least about or no more than about 130 to about 400 nucleic acid bases, including about 150 to about 400 nucleic acid bases, about 150 to about 300 nucleic acid bases, about 150 to about 200 nucleic acid bases, about 200 to about 400 nucleic acid bases, about 200 to about 300 nucleic acid bases, about 300 to about 400 nucleic acid bases, and so forth. As described herein, said size-selected plurality of DNA fragments have lengths from at least about or no more than about 30 to about 250 nucleic acid bases, about 30 to about 200 nucleic acid bases, about 30 to about 100 nucleic acid bases, about 75 to about 250 nucleic acid bases, about 75 to about 200 nucleic acid bases, about 75 to about 150 nucleic acid bases, about 75 to about 125 nucleic acid bases, about 100 to about 250 nucleic acid bases, about 100 to about 200 nucleic acid bases, about 100 to about 150 nucleic acid bases, about 175 to about 250 nucleic acid bases, about 175 to about 225 nucleic acid bases, about 200 to about 250 nucleic acid bases, and so forth.

As described herein, the method further comprises measuring a methylation status of at least a portion of said plurality of DNA fragments or at least a portion of said plurality of tagged DNA fragments, to provide a methylation profile of said at least said portion of said plurality of DNA fragments or of said size-selected plurality of tagged DNA fragments. As described herein, the method further comprises measuring a methylation status of at least a portion of said size-selected plurality of DNA fragments or at least a portion of said plurality of tagged DNA fragments, to provide a methylation profile of said at least said portion of said size-selected plurality of DNA fragments or of said plurality of tagged DNA fragments. As described herein, the method further comprises processing said methylation profile against one or more references. A methylation profile may include information (including the presence and/or absence of certain methylation sites) of any number of CpG sites, CpG-rich sequences, and/or CpG islands. As described herein, said reference comprises a reference methylation profile of cfDNA molecules of one or more additional subjects. The subject(s) from which the reference methylation profile of cfDNA is procured may be healthy, may be cancer-free, may have cancer, or may have an elevated risk for having cancer, for example.

As described herein, said plurality of cfDNA molecules is obtained from a bodily sample of said subject. As described herein, said bodily sample is selected from the group consisting of plasma, serum, bone marrow, cerebral spinal fluid, pleural fluid, saliva, stool, sputum, nipple aspirate, biopsy, cheek scrapings, urine and a combination thereof. As described herein, the method further comprises processing said cfDNA molecules from said plurality of cfDNA molecules having one or more CpG sites to generate a methylation profile for said plurality of cfDNA molecules. As described herein, the method further comprises processing said methylation profile to generate a likelihood of said subject as having or being suspected of having a disease or disorder. In cases wherein a methylation profile from a sample from an individual is compared to one or more references, the source of the sample of the one or more references may or may not be the same source as the sample of the individual.

As described herein, said disease or disorder is selected from the group consisting of cancer, multiple sclerosis, traumatic or ischemic brain damage, diabetes, pancreatitis, Alzheimer's disease, and fetal abnormality. As described herein, said disease or disorder is a cancer selected from the group consisting of pancreatic cancer, liver cancer, lung cancer, colorectal cancer, leukemia, bladder cancer, bone cancer, brain cancer, breast cancer, cervical cancer, endometrial cancer, esophageal cancer, gastric cancer, head and neck cancer, melanoma, ovarian cancer, testicular cancer, kidney cancer, sarcoma, bile duct cancer, thyroid cancer, gall bladder cancer, spleen cancer, and prostate cancer.

As described herein, the methylation patterns of cfDNA molecules, obtained from a bodily sample of said subject, can be used to monitor the abnormal tissue-specific cell death.

Described herein is a method for enriching a plurality of deoxyribonucleic acid (DNA) fragments from a plurality of cell-free DNA (cfDNA) molecules of a subject, comprising: (a) modifying one or both ends of each of at least a portion of said plurality of cell-free DNA molecules or derivatives thereof to provide a plurality of modified cell-free DNA molecules having ends that are (i) incapable of coupling with adapters or (ii) configured for separation from a remainder of said plurality of cfDNA; (b) subjecting said plurality of modified cell-free DNA molecules to conditions sufficient to fragment each of at least a subset of said modified cell-free DNA molecules at one or more CpG sites, to provide a plurality of DNA fragments, wherein at least a subset of said plurality of DNA fragments have methylated nucleic acid bases; and (c) coupling said adapters to ends of said plurality of DNA fragments to provide a plurality of tagged DNA fragments having methylated nucleic acid bases that are distinguishable from unmethylated nucleic acid bases.

As described herein, at least a subset of said plurality of DNA fragments have methylated nucleic acid bases. As described herein, the method further comprises, prior to or after (c), separating fragments of said cfDNA molecules having said ends from said plurality of DNA fragments. As described herein, said fragments are coupled to magnetic beads, and wherein said fragments are separated using magnetic separation. As described herein, in (a), ends of said modified cell-free DNA molecules are incapable of undergoing ligation or primer extension. As described herein, the method further comprises, prior to or after (c), subjecting said plurality of DNA fragments to conditions sufficient to permit said methylated nucleic acid bases to be distinguished from said unmethylated nucleic acid bases. As described herein, subjecting said plurality of DNA fragments to conditions sufficient to permit said methylated nucleic acid bases to be distinguished from said unmethylated nucleic acid bases comprises performing bisulfite conversion on said plurality of DNA fragments.

As described herein, the method further comprises, subsequent to (c), subjecting said plurality of tagged DNA fragments to conditions sufficient to permit said methylated nucleic acid bases to be distinguished from said unmethylated nucleic acid bases, thereby yielding an additional plurality of tagged DNA fragments. As described herein, subjecting said plurality of tagged DNA fragments to said conditions sufficient to permit said methylated nucleic acid bases to be distinguished from said unmethylated nucleic acid bases comprises performing bisulfite conversion on said plurality of tagged DNA fragments.

As described herein, said conditions in (b) are sufficient to fragment each of said at least said subset of said modified cell-free DNA molecules at a plurality of CpG sites. As described herein, said modifying comprises subjecting a 3' end of each of said at least said portion of said plurality of cfDNA molecules to conditions sufficient to modify said 3' end with a dideoxynucleotide (ddNTP) moiety or a functional analog thereof. As described herein, said modifying comprises subjecting a 5' end of each of said at least said portion of said plurality of cfDNA molecules to conditions sufficient to dephosphorylate said 5' end. Dephosphorylation may occur by any suitable means, including utilizing dephosphorylases such as calf intestinal alkaline phosphatase, as an example.

As described herein, said modifying comprises incorporation of one or more blocker oligonucleotides at said one or both ends of each of at least a portion of said plurality of cfDNA molecules. As described herein, (b) further comprises performing restriction enzyme digestion of said plurality of modified cell-free DNA molecules to fragment each of said at least said subset of said modified cell-free DNA molecules at said one or more CpG sites. As described herein, said restriction enzyme digestion is performed using one or more restriction enzymes that enrich for fragments having CpG sites. As described herein, said one or more restriction enzymes comprise MspI, HpaII, and/or TaqI.

As described herein, each of said adapters comprises a functional sequence that is configured to couple to a flow cell of a nucleic acid sequencer. As described herein, coupling said adapters in (c) comprises ligating said adapters to said ends of said plurality of DNA fragments. As described herein, the method further comprises, prior to said ligation, performing end repair or nucleic acid base tailing of said plurality of DNA fragments. As described herein, the method further comprises, prior to said ligation, performing end repair and nucleic acid base tailing of said plurality of DNA fragments. As described herein, the adaptors are labeled.

As described herein, said adapters are configured to be coupled to a nucleic acid molecule to provide a library for sequencing. As described herein, said adapters are configured to be ligated to said nucleic acid molecule. As described herein, said adapters comprise at least one stem-loop region. As described herein, the method further comprises coupling said adapters to said nucleic acid molecule, and linearizing said stem-loop region of said adapters coupled to said nucleic acid molecule. As described herein, said linearizing is performed using an endonuclease, a uracil glycosylase or a functional analog thereof, or a combination thereof. As described herein, said endonuclease is endonuclease VIII or a functional analog thereof. As described herein, said uracil glycosylase is a uracil deoxyribonucleic nucleic acid (DNA) glycosylase.

As described herein, said adapters are Y shaped. As described herein, said adapters are blunt ended. As described herein, said adapters comprise a known sequence. As described herein, said adapters comprise a unique sequence that allows unique molecular identification of said plurality of tagged DNA fragments or derivatives thereof.

As described herein, said nucleic acid bases of said adapters are unmethylated. As described herein, said nucleic acid bases of said adapters are methylated. As described herein, the method further comprises subjecting said plurality of DNA fragments or said plurality of tagged DNA fragments to amplification. As described herein, said amplification comprises polymerase chain reaction (PCR).

As described herein, the method further comprises performing size selection of said plurality of DNA fragments or said plurality of tagged DNA fragments to provide a size-selected plurality of DNA fragments. As described herein, said size-selected plurality of DNA fragments have lengths from about 130 to about 400 nucleic acid bases. As described herein, said size-selected plurality of DNA fragments have lengths from about 30 to about 250 nucleic acid bases.

As described herein, the method further comprises measuring a methylation status of at least a portion of said plurality of DNA fragments or said plurality of tagged DNA fragments, to provide a methylation profile of said at least said portion of said plurality of DNA fragments or said plurality of tagged DNA fragments. As described herein, the method further comprises measuring a methylation status of at least a portion of said size-selected plurality of DNA fragments, to provide a methylation profile of said at least said portion of said size-selected plurality of DNA fragments or size-selected plurality of tagged DNA fragments. As described herein, the method further comprises processing said methylation profile against one or more references.

As described herein, the method further comprises subjecting at least a portion of said size-selected plurality of DNA fragments or size-selected plurality of tagged DNA fragments or derivatives thereof to nucleic acid sequencing to yield a plurality of sequence reads. As described herein, said reference comprises a reference methylation profile of cfDNA molecules of one or more additional subjects. As described herein, said plurality of cfDNA molecules is obtained from a bodily sample of said subject. As described herein, said bodily sample is selected from the group consisting of plasma, serum, bone marrow, cerebral spinal fluid, pleural fluid, saliva, stool, sputum, nipple aspirate, biopsy, cheek scrapings and urine.

Described herein is a method for processing or analyzing a plurality of cell-free deoxyribonucleic (DNA) molecules, comprising: (a) retrieving a plurality of sequence reads generated by a sequencer, wherein at least a subset of said plurality of sequence reads comprises individual sequence reads comprising (i) sequences from said plurality of cell-free DNA molecules and (ii) adapter sequences at both ends of each of said individual sequence reads, which adapter sequences are not from said plurality of cell-free DNA molecules; (b) processing said plurality of sequence reads to (i) identify one or more sequence reads from said plurality of sequence reads having said adapter sequences at both ends, and (ii) identifying said one or more sequence reads as being associated with one or more CpG sites of said plurality of cell-free DNA molecules; and (c) using said one or more CpG sites identified in (b) to generate a methylation profile for said plurality of cell-free DNA molecules. As described herein, the methylation profile is utilized in clinical approaches for the diagnosis, prognosis, treatment efficacy, and/or treatment regimen for an individual.

As described herein, said one or more CpG sites comprise two or more, three or more, or four or more CpG sites. As described herein, the method further comprises producing a report, such as electronically outputting a report indicative of said methylation profile. As described herein, the method further comprises processing said methylation profile to generate a likelihood or risk of said subject as having or being suspected of having at least one disease or disorder. As described herein, said disease or disorder is selected from the group consisting of cancer, multiple sclerosis, traumatic or ischemic brain damage, diabetes, pancreatitis, Alzheimer's disease, and fetal abnormality. As described herein, said disease or disorder is a cancer selected from the group consisting of pancreatic cancer, liver cancer, lung cancer, colorectal cancer, leukemia, bladder cancer, bone cancer, brain cancer, breast cancer, cervical cancer, endometrial cancer, esophageal cancer, gastric cancer, head and neck cancer, melanoma, ovarian cancer, testicular cancer, kidney cancer, sarcoma, bile duct cancer, thyroid cancer, spleen cancer, gall bladder cancer, and prostate cancer.

Described herein is a system for processing or analyzing a plurality of cell-free deoxyribonucleic (DNA) molecules, comprising: a database storing a plurality of sequence reads, wherein at least a subset of said plurality of sequence reads comprises individual sequence reads comprising (i) sequences from said plurality of cell-free DNA molecules and (ii) adapter sequences at both ends of each of said individual sequence reads, which adapter sequences are not from said plurality of cell-free DNA molecules; and one or more computer processors operatively coupled to said database, wherein said one or more computer processors are individually or collectively programmed to: (1) retrieve said plurality of sequence reads from said database; (2) process said plurality of sequence reads to (i) identify one or more sequence reads from said plurality of sequence reads having said adapter sequences at both ends, and (ii) identifying said one or more sequence reads as being associated with one or more CpG sites of said plurality of cell-free DNA molecules; and (3) use said one or more CpG sites identified in (2) to generate a methylation profile for said plurality of cell-free DNA molecules. Following this, the methylation profile may indicate whether or not the individual associated with the sequence reads has a particular disease or disorder, including cancer, for example. The methylation profile may indicate whether or not the individual has a certain type of cancer, has a certain stage of cancer, will respond well to one or more specific treatments, the life expectancy of the individual, and so forth.

As described herein, said one or more CpG sites comprise two or more CpG sites. As described herein, said one or more computer processors are individually or collectively programmed to electronically output a report indicative of said methylation profile. As described herein, said one or more computer processors are individually or collectively programmed to process said methylation profile to generate a likelihood or risk of said subject as having or being suspected of having one or more diseases or disorders. As described herein, said disease or disorder is selected from the group consisting of cancer, multiple sclerosis, traumatic or ischemic brain damage, diabetes, pancreatitis, Alzheimer's disease, and fetal abnormality. As described herein, said disease or disorder is a cancer selected from the group consisting of pancreatic cancer, liver cancer, lung cancer, colorectal cancer, leukemia, bladder cancer, bone cancer, brain cancer, breast cancer, cervical cancer, endometrial cancer, esophageal cancer, gastric cancer, head and neck cancer, melanoma, ovarian cancer, testicular cancer, kidney cancer, sarcoma, bile duct cancer, thyroid cancer, spleen cancer, gall bladder cancer, and prostate cancer.

As described herein, the methylation patterns of cfDNA molecules, obtained from a bodily sample of said subject, can be used to monitor the abnormal tissue-specific cell death.

Described herein is a non-transitory computer-readable medium comprising machine executable code that, upon execution by one or more computer processors, implements a method for processing or analyzing a plurality of cell-free deoxyribonucleic (DNA) molecules, said method comprising: (a) retrieving a plurality of sequence reads generated by a sequencer, wherein at least a subset of said plurality of sequence reads comprises individual sequence reads comprising (i) sequences from said plurality of cell-free DNA molecules and (ii) adapter sequences at both ends of each of said individual sequence reads, which adapter sequences are not from said plurality of cell-free DNA molecules; (b) processing said plurality of sequence reads to (i) identify one or more sequence reads from said plurality of sequence reads having said adapter sequences at both ends, and (ii) identifying said one or more sequence reads as being associated with one or more CpG sites of said plurality of cell-free DNA molecules; and (c) using said one or more CpG sites identified in (b) to generate a methylation profile for said plurality of cell-free DNA molecules.

As described herein, there is a method of enriching a collection of CpG-rich sequences from cfDNA (including obtained from blood or plasma or urine, or a combination thereof), comprising the steps of labeling or modifying ends of cfDNA molecules to produce labeled cfDNA molecules, wherein the ends of the labeled cfDNA molecules are unable to be subject to ligation; digesting the labeled cfDNA molecules with one or more restriction enzymes (such as MspI, HpaII, TaqI, or a mixture that comprises MspI, TaqI, and/or HpaII) that recognize C^CGG, T^CGA or other sites in methylated form, unmethylated form, or both, to produce digested cfDNA molecules that are ligatable on both ends and to produce digested cfDNA molecules that are ligatable on only one end; ligating methylated adapters to the ligatable ends of the digested cfDNA molecules, thereby producing adapter-ligated cfDNA molecules; subjecting the adapter-ligated cfDNA molecules to bisulfite conversion to produce bisulfite-converted adapter-ligated cfDNA molecules; and amplifying (such as by polymerase chain reaction) the bisulfite-converted adapter-ligated cfDNA molecules that comprise adapters on both ends of the molecules. As described herein, the adapter can ligate to single-strand DNA and the bisulfite conversion can be performed before adapter ligation. As described herein, the method further comprises the step of size selecting the amplified bisulfite-converted adapter-ligated cfDNA molecules. The size selected amplified bisulfite-converted adapter-ligated cfDNA molecules may have lengths between about 150 and about 400 nucleotides. As described herein, the labeling step comprises dephosphorylation of the 5' ends of the cfDNA molecules prior to or after the labeling. The labeling may comprise adding ddNTPs to the 3' end of the cfDNA molecules, and also described herein the label is detectable. As described herein, the label comprises ddNTP that is fluorescent, colorimetric, biotinylated, radioactive, or a combination thereof. As described herein, methods further comprise a step of end repair and nucleotide tailing of the digested cfDNA molecules prior to the ligating step.

As described herein, the adapter comprises at least one stem loop region. In such cases, the method may further comprise the step of linearizing the stem loop region of the adapter on the adapter-ligated cfDNA molecules. The linearizing may be performed by at least one uracil DNA glycosylase, is performed by a restriction enzyme, or both. As described herein the linearizing is performed by a mixture of Uracil DNA glycosylase and Endonuclease VIII. Also described herein, the adapter is fork-shaped. The adapter may comprise one or more known sequences, including one or more unique sequences.

As described herein, the method further comprises the step of obtaining the cfDNA from blood or plasma. Some or all of the size selected amplified cfDNA molecules may be analyzed, for example sequenced in part or in full. Also described herein, some or all of the size selected amplified cfDNA molecules are analyzed for methylation patterns, and the methylation pattern of some or all of the size selected amplified cfDNA molecules may or may not be compared to a reference. The methylation pattern of some or all of the size selected amplified cfDNA molecules from cfDNA from a first individual may be compared to one or more methylation patterns in DNA of a second or more individual.

Methods of the disclosure include those for enriching a collection of CpG-rich sequences from cell-free DNA (cfDNA), comprising the steps of modifying ends of cfDNA molecules to produce cfDNA molecules in which ends of the labeled cfDNA molecules are unable to be subject to ligation. The modification to the ends to prevent ligation may be achieved in one or more modifications to the end. For example, the 5' ends and/or the 3' ends of the DNA may be modified. Also described herein, the 5' ends are dephosphorylated and in addition to or alternative to this, the 3' ends are modified through addition of an agent to the 3' end of the DNA, for example with a ddNTP.

### BRIEF DESCRIPTION OF THE DRAWINGS

FIG. 1 illustrates a flowchart of performing methylation profiling of cell-free DNA (cfDNA).
FIG. 2 illustrates methylation profiling of cfDNA using cell-free RRBS (cfRRBS) with Y-shaped adapters.
FIG. 3 illustrates methylation profiling of cfDNA using cell-free RRBS (cfRRBS) with stem-loop adapters.
FIG. 4 illustrates methylation profiling of cfDNA using cell-free RRBS (cfRRBS) with single-strand ligation adapters.
FIG. 5 illustrates methylation profiling of cfDNA using cell-free RRBS (cfRRBS) with removal by streptavidin magnetic beads.
FIGS. 6A-6C. FIG. 6A illustrates truncated Y-shaped adapters, FIG. 6B illustrates truncated Y-shaped adapters with barcodes at the ends, and FIG. 6C illustrates truncated Y-shaped adapters with barcodes plus nucleic acid bases left by enzymatic digestion at the ends.
FIGS. 7A-7C. FIG. 7A illustrates stem-loop adapters, FIG. 7B illustrates stem-loop adapters with barcodes at the ends, and FIG. 7C illustrates stem-loop adapters with barcodes plus nucleic acid bases left by enzymatic digestion at the ends.
FIGs. 8A-8D illustrate examples of different single-strand ligation adapters.
FIG. 9 illustrates a comparison of gel electrophoresis of products from an RRBS assay and from a cfRRBS assay.
FIG. 10 illustrates a computer system that is programmed or otherwise configured to implement methods described herein.

### DETAILED DESCRIPTION

The words "a" and "an" when used in the present specification in concert with the word comprising, including the claims, denote "one or more." The present disclosure may consist of or consist essentially of one or more elements, method steps, and/or methods of the present disclosure. It is contemplated that any method, system, or composition described herein can be implemented with respect to any other method or composition described herein.

### I. Examples of Definitions

The description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges may include the range endpoints.

The term "subject," as used herein, generally refers to an individual having a biological sample that is undergoing processing or analysis. A subject can be an animal or plant. The subject can be a mammal, such as a human, dog, cat, horse, pig or rodent. The subject can be a patient, e.g., have or be suspected of having or at risk for having a disease, such as one or more cancers (e.g., brain cancer, breast cancer, cervical cancer, colorectal cancer, endometrial cancer, esophageal cancer, gastric cancer, hepatobiliary tract cancer, leukemia, liver cancer, lung cancer, lymphoma, ovarian cancer, pancreatic cancer, skin cancer, urinary tract cancer, testicular cancer, kidney cancer, sarcoma, bile duct cancer, thyroid cancer, gall bladder cancer, spleen cancer, or prostate cancer, and the cancer may or may not comprise solid tumor(s)), one or more infectious diseases, one or more genetic disorder, or one or more tumors, or any combination thereof. For subjects having or suspected of having one or more tumors, the tumors may be of one or more types. The subject may have a disease or be suspected of having the disease. The subject may be asymptomatic.

The term "sample," as used herein, generally refers to a biological sample. The samples may be taken from tissue and/or cells or from the environment of tissue and/or cells. In some examples, the sample may comprise, or be derived from, a tissue biopsy, blood (e.g., whole blood), blood plasma, extracellular fluid, dried blood spots, cultured cells, culture media, discarded tissue, plant matter, synthetic proteins, bacterial and/or viral samples, fungal tissue, archaea, or protozoans. The sample may have been isolated from the source prior to collection. Samples may comprise forensic evidence. Non-limiting examples include a fingerprint, saliva, urine, blood, stool, semen, or other bodily fluids isolated from the primary source prior to collection. In some examples, the sample is isolated from its primary source (cells, tissue, bodily fluids such as blood, environmental samples, etc.) during sample preparation. The sample may be derived from an extinct species including samples derived from fossils. The sample may or may not be purified or otherwise enriched from its primary source. Also described herein, the primary source is homogenized prior to further processing. The sample may be filtered or centrifuged to remove buffy coat, lipids, or particulate matter. The sample may also be purified or enriched for nucleic acids, or may be treated with RNases or DNases. The sample may contain tissues and/or cells that are intact, fragmented, or partially degraded.

The sample may be obtained from a subject with a disease or disorder, and the subject may or may not have had a diagnosis of the disease or disorder. The subject may be in need of a second opinion. The disease or disorder may be an infectious disease, an immune disorder or disease, a cancer, a genetic disease, a degenerative disease, a lifestyle disease, or an injury. The infectious disease may be caused by bacteria, viruses, fungi, and/or parasites. Non-limiting examples of cancers include pancreatic cancer, liver cancer, lung cancer, colorectal cancer, leukemia, bladder cancer, bone cancer, brain cancer, breast cancer, cervical cancer, endometrial cancer, esophageal cancer, gastric cancer, head and neck cancer, melanoma, ovarian cancer, thyroid cancer, gall bladder cancer, spleen cancer, and prostate cancer. Some examples of genetic diseases or disorders include, cystic fibrosis, Charcot-Marie-Tooth disease, Huntington's disease, Peutz-Jeghers syndrome, Down syndrome, Rheumatoid arthritis, and Tay-Sachs disease. Non-limiting examples of lifestyle diseases include obesity, diabetes, arteriosclerosis, heart disease, stroke, hypertension, liver cirrhosis, nephritis, cancer, chronic obstructive pulmonary disease (COPD), hearing problems, and chronic backache. Some examples of injuries include, abrasion, brain injuries, bruising, burns, concussions, congestive heart failure, construction injuries, dislocation, flail chest, fracture, hemothorax, herniated disc, hip pointer, hypothermia, lacerations, pinched nerve, pneumothorax, rib fracture, sciatica, spinal cord injury, tendons ligaments fascia injury, traumatic brain injury, and whiplash. The sample may be taken before and/or after treatment of a subject with a disease or disorder. Samples may be taken before and/or after a treatment of the subject for a disease or disorder. Samples may be taken during a treatment or a treatment regimen. Multiple samples may be taken from a subject to monitor the effects of a treatment over time, including beginning from prior to the onset of the treatment. The sample may be taken from a subject known or suspected of having an infectious disease for which diagnostic antibodies may or may not be available.

The sample may be taken from a subject suspected of having a disease or a disorder. The sample may be taken from a subject experiencing unexplained symptoms, such as fatigue, nausea, weight loss, aches, pains, weakness, or memory loss. The sample may be taken from a subject having explained symptoms. The sample may be taken from a subject at risk of developing a disease or disorder because of one or more factors such as familial and/or personal history, age, environmental exposure, lifestyle risk factors, presence of other known risk factor(s), or a combination thereof.

The sample may be taken from a healthy individual. Also described herein, samples may be taken longitudinally from the same individual. Also described herein, samples acquired longitudinally may be analyzed with the goal of monitoring individual health and early detection of health issues (e.g., early diagnosis of cancer). As described herein, the sample may be collected at a home setting or at a point-of-care setting and subsequently transported by a mail delivery, courier delivery, or other transport method prior to analysis. For example, a home user may collect a blood spot sample through a finger prick, and the blood spot sample may be dried and subsequently transported by mail delivery prior to analysis. As described herein, samples acquired longitudinally may be used to monitor response to stimuli expected to impact health, athletic performance, or cognitive performance. Non-limiting examples include response to medication, dieting, and/or an exercise regimen. Also described herein, the individual sample is multi-purpose and allows for methylation profiling to obtain clinically relevant information but also is used for information about the individual's personal or family ancestry.

As described herein, a biological sample is a nucleic acid sample including one or more nucleic acid molecules. The nucleic acid molecules may be cell-free or substantially cell-free nucleic acid molecules, such as cell-free DNA (cfDNA) or cell-free RNA (cfRNA) or a mixture thereof. The nucleic acid molecules may be derived from a variety of sources including human, mammal, non-human mammal, ape, monkey, chimpanzee, reptilian, amphibian, or avian sources. Further, samples may be extracted from variety of animal fluids containing cell-free sequences, including blood, serum, plasma, bone marrow, vitreous, sputum, stool, urine, tears, perspiration, saliva, semen, mucosal excretions, mucus, cerebral spinal fluid, pleural fluid, amniotic fluid, and lymph fluid. The sample may be taken from an embryo, fetus, or pregnant woman. In some examples, the sample may be isolated from the mother's blood plasma. In some examples, the sample may comprise cell-free nucleic acids (e.g., cfDNA) that are fetal in origin (via a bodily sample obtained from a pregnant subject), or are derived from tissue of the subject itself.

Components of the sample (including nucleic acids) may be tagged, e.g., with identifiable tags, to allow for multiplexing of samples. Some non-limiting examples of identifiable tags include: fluorophores, magnetic nanoparticles, and nucleic acid barcodes. Fluorophores may include fluorescent proteins such as GFP, YFP, RFP, eGFP, mCherry, tdtomato, FITC, Alexa Fluor 350, Alexa Fluor 405, Alexa Fluor 488, Alexa Fluor 532, Alexa Fluor 546, Alexa Fluor 555, Alexa Fluor 568, Alexa Fluor 594, Alexa Fluor 647, Alexa Fluor 680, Alexa Fluor 750, Pacific Blue, Coumarin, BODIPY FL, Pacific Green, Oregon Green, Cy3, Cy5, Pacific Orange, TRITC, Texas Red, Phycoerythrin, Allophcocyanin, or other fluorophores. One or more barcode tags may be attached (e.g., by coupling or ligating) to cell-free nucleic acids (e.g., cfDNA) in the sample prior to sequencing. The barcodes may uniquely tag the cfDNA molecules in a sample. Alternatively, the barcodes may non-uniquely tag the cfDNA molecules in a sample. The barcode(s) may non-uniquely tag the cfDNA molecules in a sample such that additional information taken from the cfDNA molecule (e.g., at least a portion of the endogenous sequence of the cfDNA molecule), taken in combination with the non-unique tag, may function as a unique identifier for (e.g., to uniquely identify against other molecules) the cfDNA molecule in a sample. For example, cfDNA sequence reads having unique identity (e.g., from a given template molecule) may be detected based on sequence information comprising one or more contiguous-base regions at one or both ends of the sequence read, the length of the sequence read, and the sequence of the attached barcodes at one or both ends of the sequence read. DNA molecules may be uniquely identified without tagging by partitioning a DNA (e.g., cfDNA) sample into many (e.g., at least about 50, at least about 100, at least about 500, at least about 1 thousand, at least about 5 thousand, at least about 10 thousand, at least about 50 thousand, or at least about 100 thousand) different discrete subunits (e.g., partitions, wells, or droplets) prior to amplification, such that amplified DNA molecules can be uniquely resolved and identified as originating from their respective individual input molecules of DNA.

Any number of samples may be multiplexed. For example, a multiplexed analysis may contain at least about 2, about 3, about 4, about 5, about 6, about 7, about 8, about 9, about 10, about 11, about 12, about 13, about 14, about 15, about 16, about 17, about 18, about 19, about 20, about 25, about 30, about 35, about 40, about 45, about 50, about 55, about 60, about 65, about 70, about 75, about 80, about 85, about 90, about 95, about 100, or more samples. The identifiable tags may provide a way to interrogate each sample as to its origin, or may direct different samples to segregate to different areas or a solid support.

Any number of samples may be mixed prior to analysis without tagging or multiplexing. For example, a multiplexed analysis may contain at least about 2, about 3, about 4, about 5, about 6, about 7, about 8, about 9, about 10, about 11, about 12, about 13, about 14, about 15, about 16, about 17, about 18, about 19, about 20, about 25, about 30, about 35, about 40, about 45, about 50, about 55, about 60, about 65, about 70, about 75, about 80, about 85, about 90, about 95, about 100, or more samples. Samples may be multiplexed without tagging using a combinatorial pooling design in which samples are mixed into pools in a manner that allows signal from individual samples to be resolved from the analyzed pools using computational demultiplexing.

The samples may be enriched prior to sequencing. For example, the cfDNA molecules may be selectively enriched or non-selectively enriched for one or more regions from the subject's genome or transcriptome. For example, the cfDNA molecules may be selectively enriched for one or more regions from the subject's genome or transcriptome by targeted sequence capture (e.g., using a panel), selective amplification, or targeted amplification. As another example, the cfDNA molecules may be non-selectively enriched for one or more regions from the subject's genome or transcriptome by universal amplification. As described herein, amplification comprises universal amplification, whole genome amplification, or non-selective amplification. The cfDNA molecules may be size selected for fragments having a length in a predetermined range. For example, size selection can be performed on DNA fragments prior to adapter ligation for lengths in a range of about 40 base pairs (bp) to about 250 bp. As another example, size selection can be performed on DNA fragments after adapter ligation for lengths in a range of about 160 bp to about 400 bp.

As described herein, a subset of sequence reads may be removed from further analysis prior to processing reads for analysis. For example, a subset of sequence reads with an quality score of less than a predetermined threshold (e.g., 90%, 99%, 99.9%, or 99.99%) may be filtered out. A set of sequence reads from a given cfDNA sample may be corrected or normalized using the barcode sequence, the length, the quality score, the GC content, or other properties of individual sequence reads.

The term "nucleic acid," or "polynucleotide," as used herein, generally refers to a molecule comprising one or more nucleic acid subunits, or nucleotides. A nucleic acid may include one or more nucleotides selected from adenosine (A), cytosine (C), guanine (G), thymine (T) and uracil (U), or variants thereof. A nucleotide generally includes a nucleoside and at least 1, 2, 3, 4, 5, 6, 7, 8, 9, 10, or more phosphate (PO3) groups. A nucleotide can include a nucleobase, a five-carbon sugar (either ribose or deoxyribose), and one or more phosphate groups, individually or in combination.

Ribonucleotides are nucleotides in which the sugar is ribose. Deoxyribonucleotides are nucleotides in which the sugar is deoxyribose. A nucleotide can be a nucleoside monophosphate or a nucleoside polyphosphate. A nucleotide can be a deoxyribonucleoside polyphosphate, such as, e.g., a deoxyribonucleoside triphosphate (dNTP), which can be selected from deoxyadenosine triphosphate (dATP), deoxycytidine triphosphate (dCTP), deoxyguanosine triphosphate (dGTP), uridine triphosphate (dUTP) and deoxythymidine triphosphate (dTTP) dNTPs, that include detectable tags, such as luminescent tags or markers (e.g., fluorophores). A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). In some examples, a nucleic acid is deoxyribonucleic acid (DNA), ribonucleic acid (RNA), or derivatives or variants thereof. A nucleic acid may be single-stranded or double stranded. A nucleic acid molecule may be linear, curved, or circular or any combination thereof.

The terms "nucleic acid molecule," "nucleic acid sequence," "nucleic acid fragment," "oligonucleotide" and "polynucleotide," as used herein, generally refer to a polynucleotide, such as deoxyribonucleotides (DNA) or ribonucleotides (RNA), or analogs and/or combinations thereof (e.g., mixture of DNA and RNA). A nucleic acid molecule may have various lengths. A nucleic acid molecule can have a length of at least about 5 bases, 10 bases, 20 bases, 30 bases, 40 bases, 50 bases, 60 bases, 70 bases, 80 bases, 90, 100 bases, 110 bases, 120 bases, 130 bases, 140 bases, 150 bases, 160 bases, 170 bases, 180 bases, 190 bases, 200 bases, 300 bases, 400 bases, 500 bases, 1 kilobase (kb), 2 kb, 3, kb, 4 kb, 5 kb, 10 kb, or 50 kb or it may have any number of bases between any two of the aforementioned values. An oligonucleotide is typically composed of a specific sequence of four nucleotide bases: adenine (A); cytosine (C); guanine (G); and thymine (T) (uracil (U) for thymine (T) when the polynucleotide is RNA). Thus, the terms "nucleic acid molecule," "nucleic acid sequence," "nucleic acid fragment," "oligonucleotide" and "polynucleotide" are at least in part intended to be the alphabetical representation of a polynucleotide molecule. Alternatively, the terms may be applied to the polynucleotide molecule itself. This alphabetical representation can be input into databases in a computer having a central processing unit and/or used for bioinformatics applications such as functional genomics and homology searching. Oligonucleotides may include one or more nonstandard nucleotide(s), nucleotide analog(s) and/or modified nucleotides.

The terms "cell-free DNA" or "cfDNA," as used herein, generally refer to DNA that is freely circulating in fluids of a body, such as the bloodstream or plasma therefrom. The methods utilized herein, the cfDNA encompasses a particular type of cfDNA, such as circulating tumor DNA (ctDNA) that is tumor-derived fragmented DNA in the bloodstream that is not associated with cells. The cf DNA may be double-stranded, single-stranded, or have characteristics of both.

The term "CpG site," as used herein, generally refers to a position along a nucleic acid molecule that includes a cytosine (C) adjacent to a guanine (G) along a 5' to 3' direction. The nucleic acid molecule may include at least 1, 2, 3, 4, 5, 6, 7, 8, 9, 10, 20, 30, 40, 50, 100, 500, 1000, 10000, or more CpG sites. Such a CpG site along the 3' to 5' direction of a nucleic acid molecule may be referred to as a "GpC site."

The term "CpG island," as used herein, generally refers to a contiguous region of genomic DNA that satisfies the criteria of (1) having a frequency of CpG dinucleotides corresponding to an "observed-to-expected ratio" greater than about 0.6; (2) having a "GC Content" greater than about 0.5; and (3) having a length of at least about 0.2 kilobases (kb), with the possible exception that repeat regions matching these criteria are excluded or masked. Criteria for identifying CpG islands are described by, for example, Gardiner-Garden et al. (J. Mol. Biol., 196:262-282, 1987).

The term "CpG-rich," as used herein, generally refers to genomic regions that have high CpG content, where the majority of DNA methylation may occur. Regions of high CpG content may have a CpG content of at least 1%, 2%, 3%, 4%, 5%, 6%, 7%, 8%, 9%, 10%, or greater. Also described herein, such CpG content is greater than 1%. As described herein, CpG-rich regions may comprise CpG islands and promoter regions. CpG-rich regions may include any length (e.g., without a length restriction to be at least 0.2 kb).

The term "bisulfite conversion," as used herein, generally refers to a biochemical process for converting unmethylated bases (e.g., cytosine bases) to uracil bases, whereby based (e.g., methylated cytosine) are preserved. Examples of reagents for bisulfite conversion include sodium bisulfite, magnesium bisulfite, and trialkylammonium bisulfite.

### II. Enrichment of DNA with CpG-rich regions

The present disclosure describes efficient enrichment of particular DNA having CpG-rich regions, including cfDNA that may be double-stranded or single-stranded or have characteristics of both and the enrichment allows subsequent analysis or analyses to be more efficient. Described herein are useful methods, systems, and compositions for obtaining information concerning methylation of cfDNA that may be used clinically for screening, diagnosis, prognosis, or treatment for a particular individual. The individual may have or be suspected of having or require a treatment plan for a particular disease or disorder, and the present disclosure encompasses cancer and non-cancer disclosures.

Cancer cells may display aberrant DNA methylation patterns. Hyper-methylated and/or hypo-methylated tumor DNA fragments can be released into the bloodstream *via* processes such as cell apoptosis or necrosis, where they may become part of the circulating cell-free DNA (cfDNA) in bodily fluids, such as plasma or urine. Thus, cfDNA methylation profiling is a useful strategy for cancer screening or screening for other diseases or disorders. Whole genome bisulfite sequencing provides a comprehensive view of the DNA methylome, but it can be expensive to deep sequence the entire genome.

Reduced Representation Bisulfite Sequencing (RRBS) may be performed as a cost-effective technique for the methylation profiling of genome regions that have high CpG content, or CpG sites. Such CpG sites may be of interest because most DNA methylation occurs at CpG sites. In RRBS, genomic DNA may be digested with a restriction endonuclease (such as MspI restriction enzyme) to produce fragments, and the fragments may be size-selected to enrich for fragments with CpG-dense regions. These regions may comprise a small portion (about 3%) of the genome but provides comprehensive DNA methylation information about the genome. The fragmented nature of cell-free DNA, which may exhibit a characteristic peak around 166 base pairs (bp), may pose challenges for typical RRBS. Performing RRBS on cell-free DNA fragments to select fragments in a particular size range (such as those between 40 and 220 bp) may select all or nearly all the population of cfDNA and hence result in low enrichment.

While most fragments generated from genomic DNA and present in a typical RRBS library may have been cut twice by the restriction endonuclease (e.g., MspI), this may not hold true for cell-free DNA due to the fragmented nature of cell-free DNA. Therefore, performing typical RRBS on cell-free DNA may lead to challenges owing to limited CpG enrichment. Methods and systems to enrich cell-free DNA molecules for CpG-rich regions may advantageously allow methylation profiling toward clinical diagnostic applications.

Described herein are novel techniques for the enrichment of CpG islands in cfDNA. Described herein are cost-effective methylation profiling, and the methods described herein are useful for cancer diagnosis, including early diagnosis with liquid biopsy, for example.

Described herein are methods, compositions, and systems for evaluating DNA methylation in CpG-rich regions of cell-free DNA molecules. Methods, compositions, and systems to enrich cell-free DNA molecules for CpG-rich regions may advantageously allow methylation profiling toward clinical diagnostic applications. Described herein are improved methods and systems enriching cfDNA molecules for CpG-rich regions, including facilitating preparation of libraries from one or more samples of cfDNA for methylation profiling, such as blood-borne samples or plasma-borne samples or urine-borne samples (or a combination thereof).

Described herein are methods, compositions, and systems for preparation of molecules for analysis of methylation amounts and/or locations in the molecules. Described herein, the molecules comprise cfDNA. Described herein, the cfDNA is obtained from or derived from one or more bodily samples of a subject. For example, the bodily sample may be blood, plasma, serum, bone marrow, cerebral spinal fluid, pleural fluid, saliva, stool, nipple aspirate, cheek scrapings, sputum, and/or urine samples from the subject.

Cancer cells can display aberrant DNA methylation patterns, such as hypermethylation of one or more regulatory regions (including promoter region(s)) of one or more tumor suppressor genes and pervasive hypomethylation of one or more intergenic regions. Therefore, the DNA methylation profile of a subject or patient can be processed as a target for evaluation in clinical practice, including cancer evaluation. Hypermethylated and/or hypomethylated tumor DNA fragments can be released into the bloodstream via processes such as cell apoptosis or necrosis, where these circulation tumor DNA (ctDNA) can become part of the circulating cell-free DNA (cfDNA) in plasma. The minimally invasive or non-invasive nature of cfDNA methylation profiling may render such cfDNA methylation profiling an effective strategy for general screening or diagnosis, prognosis, treatment selection, or treatment monitoring for any disease or disorder, including for cancer. Described herein are methods and systems for processing or enriching cfDNA molecules for genomic regions that are informative for methylation profiling, such as CpG islands, such that the processed methylation profiling may be performed more effectively as compared to methylation profiling in the absence of such processing or enrichment. Described herein, the subject has or is suspected of having or is at risk of having a disease or disorder (e.g., cancer), and processing the cfDNA molecules to perform methylation profiling may help determine a likelihood of whether the subject has or is suspected of having or is at risk of having cancer.

In methods for non-invasive screening, including for cancer screening and identification of the tumor tissue-of-origin, detection and characterization of cell-free DNA from bodily samples of a subject (e.g., in plasma, blood, and/or urine samples) can be an effective method. Liquid biopsy (which may also be referred to as fluid biopsy or fluid phase biopsy) methods, which may include a blood draw, unlike traditional tissue biopsy, is useful to diagnose a variety of different malignancies.

The present disclosure relates to processing or enriching of CpG islands in cell-free DNA such that methylation profiling is particularly effective for providing methylation information from a bodily sample of a subject. Described herein are methods of evaluating DNA methylation in CpG-rich regions of cfDNA.

The present disclosure describes methods and systems that are performed on cell-free DNA molecules rather than genomic DNA molecules. Such a distinction may distinguish methods and systems that are suitable for processing or enriching of genomic DNA for CpG islands from methods and systems that are less effective for processing or enriching cell-free DNA for CpG islands. The present disclosure describes methods and systems that improve methods for performing methylation analysis of genomic DNA to facilitate methylation analysis of cfDNA, which may have differences from genomic DNA.

The present disclosure describes improved methods and systems comprising adaptations of reduced representation bisulfite sequencing (RRBS), an efficient and high-throughput technique used to process and analyze genome-wide methylation profiles at a single nucleotide level. The RRBS technique may use a combination of restriction enzymes and bisulfite sequencing to enrich for the areas of the genome that have a high CpG content, thereby reducing an amount of DNA molecules or nucleotides to be processed for sequence analysis. Described herein, RRBS methods for enriching genomic DNA molecules may be adapted for suitability or compatibility with processing cfDNA molecules.

Described herein are methods which may be referred to as cell-free Reduced Representation Bisulfite Sequencing (cfRRBS) and which may include an RRBS-analog approach for cost-effective methylation profiling of cfDNA. Described herein, conventional RRBS methods may be modified or adapted for application to cell-free DNA, including performing modifications of 3'-ends and/or 5'-ends of cfDNA molecules or fragments to block ligation and/or polymerase extension at such ends or designed for the easy removal of cfDNA molecules, such as dideoxynucleotide (ddNTP) or biotin labeling of cfDNA, performing enzymatic digestion of cfDNA molecules (e.g., using enzymes such as MspI) to produce DNA fragments, and constructing a library from the DNA fragments. The library may be subjected to size selection for a particular range of lengths, such as 150 bp to 400 bp. In some methods and systems of the present disclosure, DNA fragments containing no or only one enzymatically recognizable sequence are discarded such that only fragments containing two or more enzymatically recognizable sequences are enriched. Described herein, such a process enriches for molecules comprising at least one CpG site, thereby facilitating cost-effective sequencing for broad clinical application of screening and diagnosis tools.

The disclosure described herein includes enriching a collection of cell-free DNA (cfDNA) molecules for regions that comprise CpG islands. Described herein are methods of enriching a collection of CpG enriched (such as CpG island-comprising) sequences from cell-free DNA.

Described herein are methods for the analysis of cytosine methylation profiles in cfDNA samples. Methods for the detection of cytosine methylation in cfDNA samples are encompassed herein.

A cell-free DNA sample(s) from a subject may be subjected to methylation profiling for screening, diagnosis, prognosis, treatment selection, or treatment monitoring, for example of a tumor or of non-solid cancers. For example, studies may suggest that patients with certain methylation profiles may respond best to surgery, chemotherapy, radiation therapy, targeted therapy, hormone therapy, immunotherapy, or a combination thereof. An accurate methylation profiling of cfDNA samples may prevent potentially ineffective treatments from being prescribed and administered to patients.

In addition, one or more cancer treatments may be prescribed and administered to patients based at least in part on a methylation profile in the patient. Methods of performing methylation profiling in patients may comprise genomic DNA analysis from tissue. For example, polymerase chain reaction (PCR) and fragment analysis of genomic DNA from normal and/or tumor tissue samples may be performed at each of a set of genetic loci to perform methylation profiling. Such methods of methylation profiling may require an availability of tumor tissue for analysis. Also described herein, the availability of tumor tissue may pose challenges. Tissue can be time-consuming and costly to retrieve, requiring coordination with pathologists. Biopsied tissue can be difficult if not impossible to obtain in some cases, can be costly and involve painful procedures, and can yield low to moderate clinical relevance due to potential cancer genome evolution. Also described herein, a patient's methylation profiling may not be available until several months or even years after an initial cancer diagnosis. Therefore, a liquid biopsy approach for performing methylation profiling may offer advantages of an earlier, less invasive, and less costly alternative to tumor biopsy.

Performing methylation profiling may be relatively straightforward when a significant portion of a bodily sample obtained from a subject is derived from tumor cells. However, in a cell-free DNA (cfDNA) sample derived from a blood sample, the detection of tumor DNA from the cfDNA and the assessment of methylation profiling therefrom may be an insensitive and noisy process. Detection of tumor DNA and assessment of methylation profiling from such insensitive and/or noisy signals may be challenging due to the overwhelming signal from non-tumor DNA (e.g., from genomic DNA from cells that are not tumor derived). Described herein are methods and systems for performing methylation profiling from cell-free DNA (cfDNA) molecules in an efficient and high-throughput manner. After enriching cfDNA molecules for fragments with CpG-rich regions, the enriched fragments may be sequenced and processed using bioinformatics approaches to obtain a methylation profile of the subject.

Described herein is a method for processing or analyzing a plurality of cell-free deoxyribonucleic (DNA) molecules of a subject, comprising: (a) subjecting said plurality of cell-free DNA (cfDNA) molecules having ends that are incapable of coupling with adapters or designed for the easy removal of cfDNA molecules, to conditions sufficient to fragment at least a subset of said cell-free DNA molecules to generate fragments that contain one or more CpG sites, to provide a plurality of DNA fragments; coupling said adapters to ends of said plurality of DNA fragments to provide a plurality of tagged DNA fragments having methylated nucleic acid bases that are distinguishable from unmethylated nucleic acid bases; (b) subjecting said plurality of tagged DNA fragments or derivatives thereof to nucleic acid sequencing to yield a plurality of sequence reads; and (c) processing said plurality of sequence reads to (i) identify sequences from said adapters at both ends of said plurality of sequence reads, and (ii) upon identifying said sequences, identifying cell-free DNA molecules from said plurality of cell-free DNA molecules as having one or more CpG sites.

Described herein is a method for enriching a plurality of deoxyribonucleic acid (DNA) fragments from a plurality of cell-free DNA (cfDNA) molecules of a subject, comprising: (a) modifying one or both ends of each of at least a portion of said plurality of cell-free DNA molecules or derivatives thereof to provide a plurality of modified cell-free DNA molecules having ends that are incapable of coupling with adapters or designed for the easy removal of cfDNA molecules; (b) subjecting said plurality of modified cell-free DNA molecules to conditions sufficient to fragment each of at least a subset of said modified cell-free DNA molecules to generate fragments that contain one or more CpG sites, to provide a plurality of DNA fragments; and (c) coupling said adapters to ends of said plurality of DNA fragments to provide a plurality of tagged DNA fragments having methylated nucleic acid bases that are distinguishable from unmethylated nucleic acid bases.

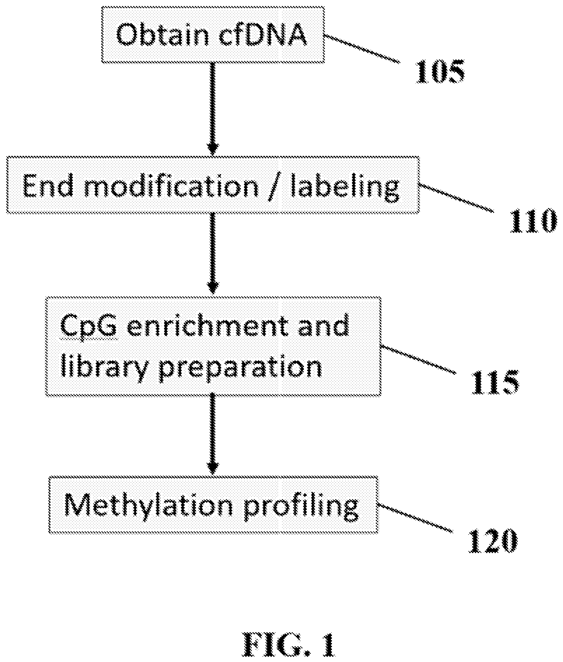

**FIG. 1** illustrates a flowchart of performing methylation profiling of cell-free DNA (cfDNA). In operation 105, a plurality of cell-free DNA (cfDNA) molecules may be obtained from a subject. Next, in operation 110, one or both ends of the plurality of cfDNA molecules may be subjected to modification or labeling to produce modified cfDNA molecules. Next, in operation 115, at least a portion of the modified cfDNA molecules may be enriched for CpG-rich regions, and libraries may be prepared from the enriched cfDNA. Next, in operation 120, methylation profiling (e.g., bisulfite sequencing) may be performed using the prepared libraries. Described herein, all operations are performed by the same entity, whereas in other cases not all of the operations are performed by the same entity. For example, operations 110 and 115 may be performed by the same entity, whereas operations 105 and 120 may be performed by an entity different from the one that performs operations 110 and 115. In other cases, operations 110, 115, and 120 are performed by the same entity

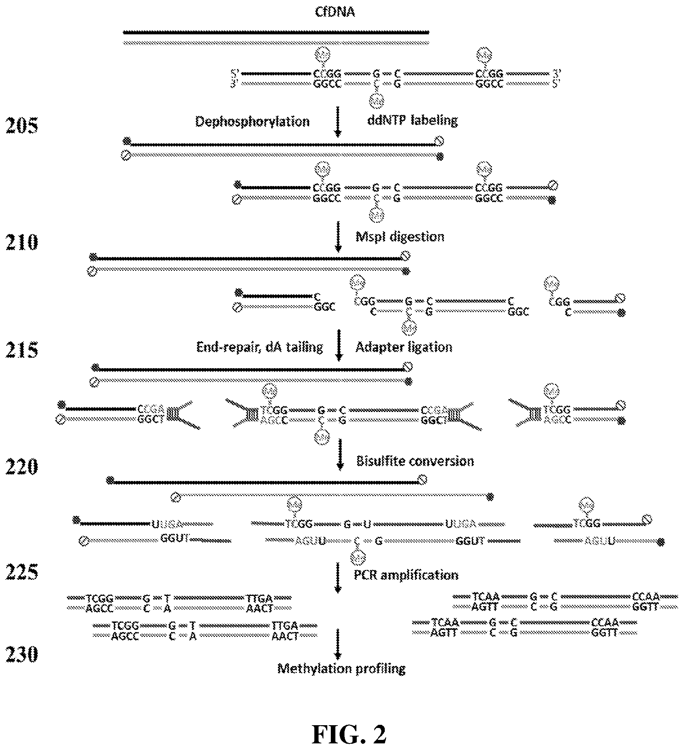

**FIG. 2** illustrates methylation profiling of cfDNA using cell-free RRBS (cfRRBS) with Y-shaped adapters. In operation 205, one or both ends of the cfDNA molecules may be modified (e.g., with a blocking group). The cfDNA molecules may be modified so as to prevent one or more subsequent activities (e.g., adapter ligation) from being performed on the modified cfDNA molecules. Ends of the cfDNA molecules may be modified by dephosphorylating the 5' ends of the cfDNA (for example, using a phosphatase, such as calf intestinal alkaline phosphatase). The dephosphorylation of 5' ends may prevent adapter ligation, for example, of the modified cfDNA molecules.

The ends of the cfDNA molecules may be modified by addition of an agent to the 3' ends of the cfDNA. The 3' ends of the cfDNA molecules may be modified with a dideoxynucleotide (ddNTP) moiety or a functional analog thereof. The ddNTP moiety may contain a label that is either detectable (such as a fluorescent signal, ion signal, colorimetric signal, biotinylated signal, or radioactive signal). Described herein, neither the dephosphorylated 5' ends nor the ddNTP-modified 3' ends of the cfDNA molecules are able to be coupled or ligated to adapters. Performing the modifications comprising 5'-end dephosphorylation, 3'-end ddNTP modification (e.g., labeling), or a combination thereof may produce cfDNA molecules having ends that are incapable of coupling with adapters. Described herein, modifications of one or both ends of cfDNA molecules prevent cfDNA fragments containing zero or one restriction enzyme (e.g., MspI) digestion site from being coupled to (e.g., ligated to) adapters. Such an action will increase the chances of enriching for the desired molecules.

The cfDNA molecules may be modified by incorporating one or more blocker oligonucleotides at one or both ends of the cfDNA molecules. For example, such blocker oligonucleotides may be PCR blocker oligonucleotides. Examples of blocker oligonucleotides include 3'-Phosphat or 3'-Inverted End (as provided by biomers.net, for example). The cfDNA ends may be biotinylated, and biotin-labeled fragments may be washed away or otherwise excluded using conjugates or supports based on avidin and/or streptavidin proteins and/or beads, including beads coated with avidin and/or streptavidin. Because of such modifications to one or both ends of the cfDNA molecules, in subsequent operations following restriction enzyme digestion, only those fragments without modified ends are able to be coupled to or ligated to adapters or will not be washed away. Such modifications may ensure that only fragments with the restriction enzyme digestion site on both ends are efficiently amplified in the prepared library.

After one or both ends of the cfDNA molecules are modified, in operation 210, the modified cfDNA molecules may be subjected to restriction enzyme digestion, thereby producing cfDNA fragments such that only those fragments without modified ends are able to be coupled to or ligated to adapters. For such cfDNA fragments, only those fragments with the restriction enzyme digestion site on both ends may be efficiently amplified in the prepared library. The modified cfDNA molecules may be digested with a restriction enzyme. The restriction enzyme may be capable of digesting DNA near CpG sites (such as CACG sites) that are either in methylated form, unmethylated form, or both. Examples of restriction enzymes include MspI, HpaII, TaqI, or others, or a mixture thereof. The restriction enzyme digestion may fragment the modified cfDNA molecules at one or more CpG sites, thereby generating two types of fragments: those with a modified (e.g., ddNTP-modified) end (e.g., a 3' end or a 5' end) on one end of the fragment and those with no modified (e.g., labeled) ends on either end of the fragment.

Prior to adapter ligation, in operation 215, the restriction enzyme-digested (e.g., MspI-digested) cfDNA fragments may be subjected to modification such that the adapters will couple or ligate thereto. For example, the ends of the MspI-digested fragments that lack modification on both ends may be modified with one or more particular nucleotides, such that the modified ends are capable of binding to one or more particular complementary nucleotides on the adapters. For example, the MspI-digested DNA fragments may be subjected to end repair and/or nucleic acid base (e.g., dNTP) tailing. Those cfDNA fragments having ddNTP-modified ends may be unable to be tailed with a dNTP, therefore such fragments may be incapable of having adapters coupled or ligated thereto.

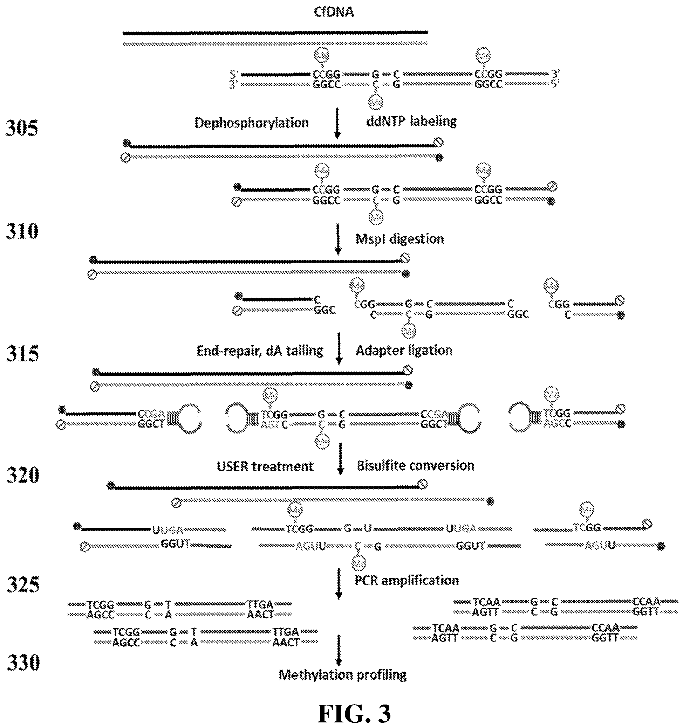

In operation 215, adapters are coupled to or ligated to the restriction enzyme-digested DNA fragments. The adapters may be of any suitable type for coupling to DNA fragments (e.g., ligation adapters). Described herein, the adapters are methylated, thereby allowing them to be unaffected by a subsequent operation to subject DNA fragments to conditions sufficient to permit said methylated nucleic acid bases to be distinguished from said unmethylated nucleic acid bases (e.g., by bisulfite conversion). Described herein, the adapters are unmethylated. The adapters may or may not have particular secondary or tertiary structure. The adapters may be generated by the user or another party, or they may be commercially obtained. The adapters may comprise one or more structures, such as forks (e.g., Y-shaped adapters or loop adapters). For example, **FIG. 2** illustrates methylation profiling of cfDNA using cell-free RRBS (cfRRBS) with Y-shaped adapters. The adapters may comprise stem loops, For example, **FIG. 3** illustrates methylation profiling of cfDNA using cell-free RRBS (cfRRBS) with stem-loop adapters.

The DNA fragments that are ddNTP-modified (e.g., have a ddNTP label) on one end may have an adapter coupled or ligated to only one end. Described herein, the adapters comprise a known sequence, which may be used in later processing steps, e.g., as a target site for primers for amplification. The adapters may have any suitable length, such as at least about 20, at least about 25, at least about 30, at least about 35, at least about 40, at least about 45, at least about 50, at least about 55, at least about 60, at least about 65, at least about 70, at least about 75, at least about 80, at least about 85, at least about 90, at least about 95, or at least about 100 bp on each side of DNA of the adapter.

In cases where the adapters comprise one or more stem loops, after performing the modification (in operation 305), the restriction-enzyme digestion (in operation 310), and the end repair and/or dA tailing and the adapter ligation (in operation 315), the adapter-ligated DNA fragments may be digested in operation 320, using one or more enzymes that linearize the stem loop such as a restriction enzyme or USER^{™} (Uracil-Specific Excision Reagent) Enzyme, thereby generating a single-nucleotide gap at the location of a uracil residue (U). Described herein, the linearizing is performed using an endonuclease, a uracil glycosylase or a functional analog thereof, or a combination thereof. Described herein, the endonuclease is endonuclease VIII or a functional analog thereof. Described herein, the uracil glycosylase is a uracil deoxyribonucleic nucleic acid (DNA) glycosylase. The USER^{™} Enzyme may be a mixture of Uracil DNA glycosylase (UDG) and the DNA glycosylase-lyase Endonuclease VIII, which is available from New England BioLabs. Described herein, an alternative to USER^{™} is Uracil-DNA excision mix (Epicentre), in which an Uracil-DNA excision mix consists of two enzymes, HK^{™}-UNG (Heat-Killable Uracil N-Glycosylase [UNG] ) and Endonuclease IV. The HK-UNG is configured to cleave the uracil base from a uracil-deoxynucleotide in DNA molecules, creating an abasic site at the location of dUTP incorporation, at which Endonuclease IV subsequently cleaves the phosphodiester bond at the abasic site. Use of the one or more enzymes used to linearize the stem loop may facilitate separation of the two strands of DNA in preparation for the subsequent bisulfite conversion (in operation 320), amplification (in operation 325), and methylation profiling (in operation 330, as described elsewhere herein).

In operation 220, the adapter-ligated DNA fragments may be subjected to conditions sufficient to permit the methylated nucleic acid bases to be distinguished from said unmethylated nucleic acid bases. Such conditions may be applied, for example, by bisulfite conversion, which converts unmethylated cytosine residues (nucleic acid bases) to uracil residues (nucleic acid bases), but does not affect methylated cytosine residues. Examples of bisulfites suitable for use in bisulfite conversion include sodium bisulfite, magnesium bisulfite, and trialkylammonium bisulfite, and one or a combination thereof may be employed.

Following bisulfite conversion, in operation 225, the bisulfite-converted DNA molecules may be subjected to amplification. The DNA molecules may be selectively or non-selectively amplified. For example, the DNA molecules may be selectively enriched for one or more regions from the subject's genome or transcriptome by selective amplification or targeted amplification. As another example, the DNA molecules may be non-selectively amplified by universal amplification, whole genome amplification, or non-selective amplification. Any kind of amplification may be performed, including polymerase chain reaction (PCR). Described herein, the amplification may use a known sequence from the adapters as targets for primers for the amplification. Described herein, amplification may be significantly dependent on coupled or ligated adapters on both ends of DNA molecules, such that only those fragments having adapters on both ends are efficiently amplified, while other fragments having adapters on zero or one end are significantly less efficiently amplified, thereby enriching the collection of DNA molecules for cfDNA fragments having restriction enzyme (e.g., MspI) digestion sites on both ends and that also include CpG islands. In operation 230, the amplified fragments may then be subsequently subjected to methylation profiling, as described elsewhere herein.

The DNA molecules may be subjected to amplification after bisulfite conversion but before size selection. When DNA molecules are subjected to amplification, only molecules having adapters coupled to or ligated to both ends may be able to be amplified efficiently. For those molecules having only have zero or one adapter coupled to or ligated to one end, the amplification may be much less efficient, thereby resulting in a negligible product. Hence, described herein, the prepared library may contain only fragments with sequences that have restriction enzyme (e.g., MspI) digestion sites on both ends, which have adapters coupled to or ligated to both ends. In such cases, the prepared library may comprise CpG-enriched DNA regions therein (e.g., CpG-rich regions and/or CpG islands). Described herein, fragments with restriction enzyme (e.g., MspI) digestion sites on both ends and that have an original DNA length (prior to coupling to or ligation to adapters) within a predetermined range (e.g., about 40 bp to about 220 bp) may be CpG-rich regions.

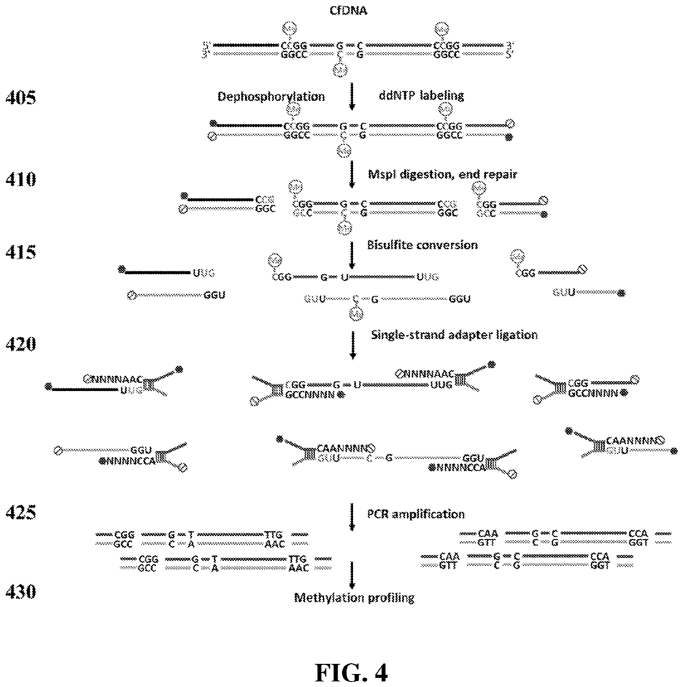

Described herein, the adapters may comprise double-strand or single-strand ligation adapters. For example, **FIG. 4** illustrates methylation profiling of cfDNA using cell-free RRBS (cfRRBS) with single-strand ligation adapters. As shown in **FIG. 4****,** when such single-strand ligation adapters are used in a method or system to enrich CpG-rich regions of cfDNA, the bisulfite conversion may be performed after the restriction enzyme (e.g., MspI) digestion and end repair, but prior to the adapter ligation. For example, the cfDNA molecules may be subjected to modification of one or both ends (in operation 405), restriction-enzyme digestion and/or end repair (in operation 410), bisulfite conversion (in operation 415), and single-strand adapter ligation (in operation 420). This order of steps may be distinguished from the case of using blunt end, Y-shaped adapters, or stem-loop adapters in a method or system to enrich CpG-rich regions of cfDNA, in which bisulfite-conversion is performed on molecules that have been restriction enzyme digested and adapter-ligated. Described herein, single-strand ligation adapters may be methylated or unmethylated. Single-strand ligation adapters may be configured to be ligatable to bisulfite-converted DNA fragments (e.g., having uracil residues in place of originally unmethylated cytosine residues) having unmodified ends (e.g., ends which are capable of being coupled or ligated thereto). Then, the adapter-ligated fragments having single-strand ligation adapters may be subjected to subsequent amplification (in operation 425) and methylation profiling (in operation 430).

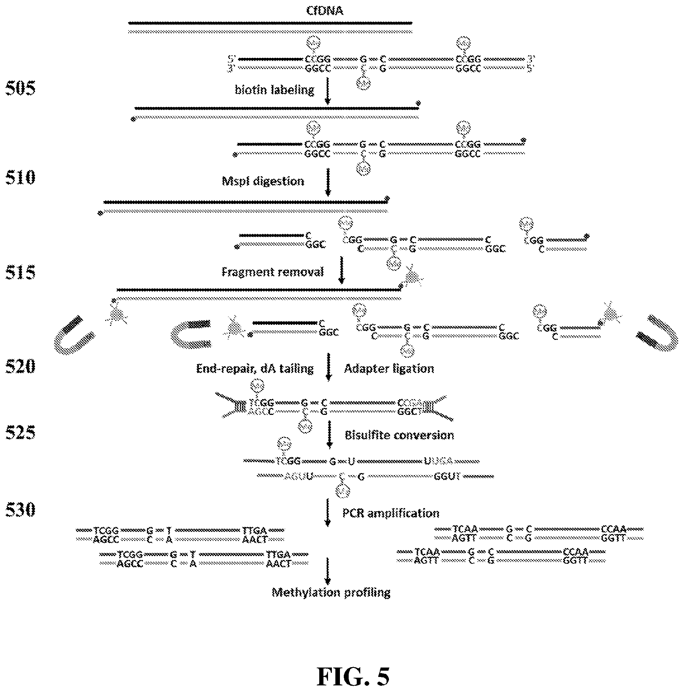

Described herein, end-modified (e.g., end-labeled, such as biotin-labeled) cell-free DNA molecules and fragments are removed or separated (e.g. by streptavidin magnetic beads) in the library preparation after restriction enzyme digestion (e.g., by MspI). For example, **FIG. 5** illustrates methylation profiling of cfDNA using cell-free RRBS (cfRRBS) with removal by streptavidin magnetic beads. The cfDNA molecules may be subjected to modification of one or both ends (in operation 505) and MspI (or other restriction enzyme) digestion (in operation 510). The fragments that have been end-modified and MspI-digested, and cell-free DNA molecules that have been end-modified but not MspI-digested, can be removed using magnetic removal (e.g., by streptavidin magnetic beads). The remaining fragments can subsequently be subjected to end repair and/or dA tailing and adapter ligation (in operation 520), bisulfite conversion (in operation 525), amplification (in operation 530), and methylation profiling (in operation 535). Alternatively, such removal of end-modified fragments can be performed after the adapter ligation, such that biotin-dNTP instead of biotin-ddNTP can be used, in which case the end modification may be unnecessary to block adapter ligation.

The adapters may comprise one or more barcodes that allows for unique molecular identifiers of cfDNA molecules. In such cases, the adapters with one or more barcodes may be blunt end, stem-looped, or fork-shaped (Y-shaped) adapters. If stem-looped adapters are utilized without molecular barcodes, then the adapters have a common sequence that is not unique within the collection of adapters. If a fork-shaped adapter is utilized without molecular barcodes, then the adapters have a common sequence that is unique within the collection of adapters. In cases wherein molecular barcodes are utilized, then there will be many more unique sequences, regardless whether it is blunt end, stem-looped or fork-shaped (Y-shaped) adapter. Described herein, the molecular barcodes are a collection of barcodes with same and different sequences. For barcodes that have the same sequence, there should be a reasonable quantity to label multiple DNA molecules.

Described herein, sample barcodes are used for library preparation. In an example, sample indexing may be performed using a set of 12 unique indexing primers (containing 12 sample barcodes) for PCR amplification, such that when different samples are to be indexed or barcoded, different indexing primers containing different sample barcodes for different samples (e.g., choosing index/barcode #5 for sample #1, choosing index/barcode #7 for sample #2, and so on) can be used. In this manner, when considering samples for subsequent sequencing, different samples can be pooled together to perform multiplexed sequencing, thereby achieving savings in cost and time. However, because of the sample barcodes, the sequencing reads can be used to indicate and distinguish which reads originated from which sample.

In cases wherein stem-looped adapters are used, barcodes may be designed in the indexing primer (for PCR amplification of the library) rather than the adapter sequence. In such cases, adapter sequences may comprise a common sequence. In cases wherein fork-shaped (Y-shaped) adapters are used, barcodes may be designed in the adapter sequence, and the primer sequence (for PCR amplification of the library) may comprise a common sequence.

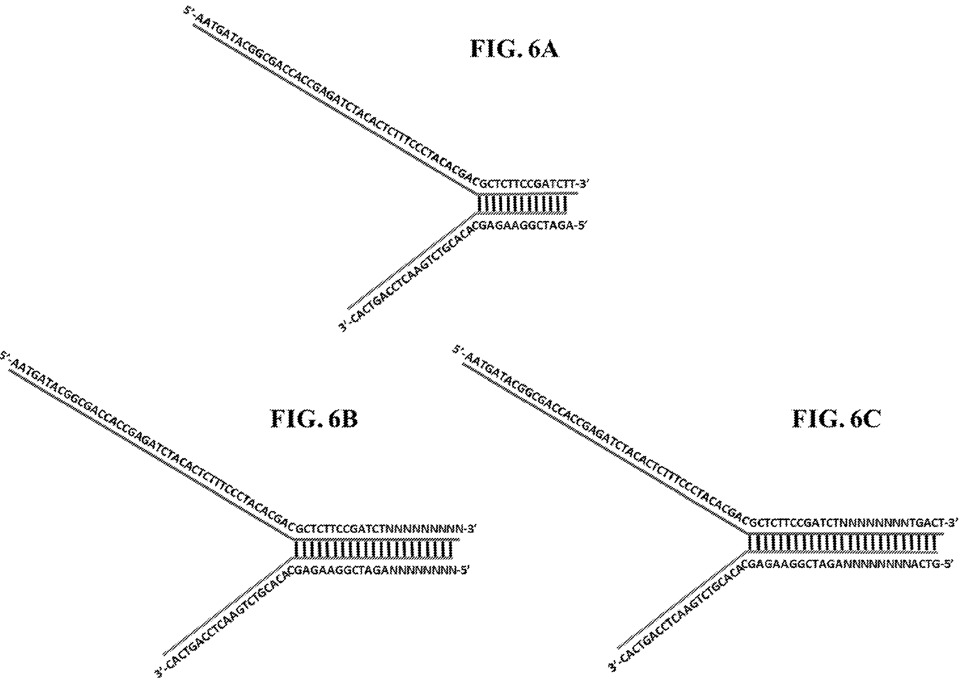

**FIG. 6A** illustrates one example of a truncated Y-shaped adapter, **FIG. 6B** illustrates one example of a truncated Y-shaped adapter with a barcode at the end, and **FIG. 6C** illustrates one example of a truncated Y-shaped adapter with barcodes plus nucleic acid bases left by enzymatic digestion at the ends. As shown in **FIG. 6B****,** Y-shaped adapters may have barcodes at their double-stranded ends, as indicated by the string of "NN...N" random nucleic acid bases (e.g., "A", "T", "C", or "G"), such that the double-stranded end is extended in length (e.g., by about 2, about 3, about 4, about 5, about 6, about 7, about 8, about 9, about 10, about 15, about 20, about 25, about 30, about 35, about 40, about 50, or more than about 50 nucleic acid bases) as compared to the Y-shaped adapter without barcodes (**FIG. 6A**). As shown in **FIG. 6C****,** Y-shaped adapters may have barcodes plus nucleic acid bases left by enzymatic digestion at the ends, such that the double-stranded end is extended in length (e.g., by about 2, about 3, about 4, about 5, about 6, about 7, about 8, about 9, or about 10 nucleic acid bases) as compared to the Y-shaped adapter without barcodes (**FIG. 6A**) and to the Y-shaped adapter with barcodes but without enzymatic digestion sites (**FIG. 6B**) and with barcodes with nucleic acid bases left by enzymatic digestion (**FIG. 6C**).

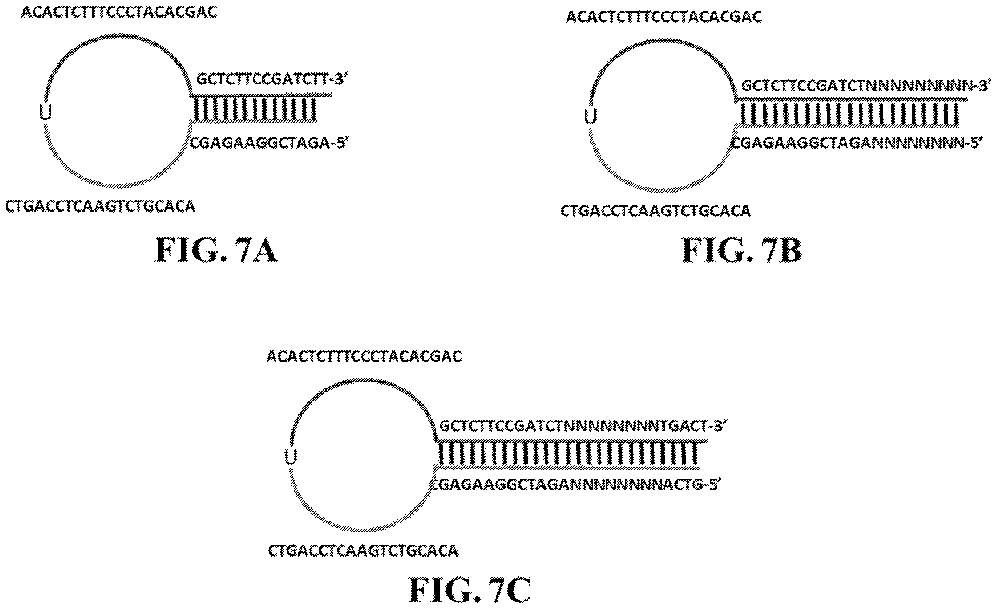

**FIG. 7A** illustrates one example of a stem-loop adapter, **FIG. 7B** illustrates one example of a stem-loop adapter with a barcode at the ends, and **FIG. 7C** illustrates one example of a stem-loop adapter with barcodes plus nucleic acid bases left by enzymatic digestion at the ends. As shown in **FIG. 7B****,** stem-loop adapters may have barcodes at their double-stranded ends, as indicated by the string of "NN... N" random nucleic acid bases (e.g., "A", "T", "C", or "G"), such that the double-stranded end is extended in length (e.g., by about 2, about 3, about 4, about 5, about 6, about 7, about 8, about 9, about 10, about 15, about 20, about 25, about 30, about 35, about 40, about 50, or more than about 50 nucleic acid bases) as compared to the stem-loop adapter without barcodes (**FIG. 7A**). As shown in **FIG. 7C****,** stem-loop adapters may have barcodes plus nucleic acid bases left by enzymatic digestion at the ends, such that the double-stranded end is extended in length (e.g., by about 2, about 3, about 4, about 5, about 6, about 7, about 8, about 9, or about 10 nucleic acid bases) as compared to the stem-loop adapter without barcodes (**FIG. 7A**) and to the stem-loop adapter with barcodes but without nucleic acid bases left by enzymatic digestion (**FIG. 7B**). Nucleic acid bases left by enzymatic digestion (**FIG. 7C**) are part of enzyme digestion sites that remain after being digested by, for example, a restriction enzyme.

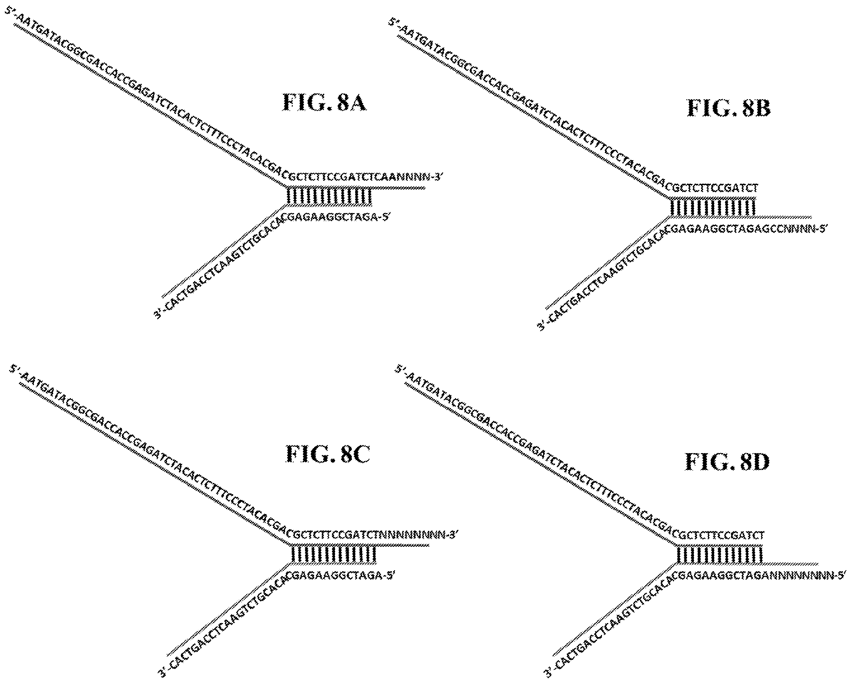

**FIGs. 8A-8D** illustrate examples of different single-strand ligation adapters. Single-strand ligation adapters may have extensions at their double-stranded ends, as indicated by the string of "NN...N" random nucleic acid bases (e.g., "A", "T", "C", or "G"), such that the double-stranded end is extended in length (e.g., by about 2, about 3, about 4, about 5, about 6, about 7, about 8, about 9, about 10, about 15, about 20, about 25, about 30, about 35, about 40, about 50, or more than about 50 nucleic acid bases).

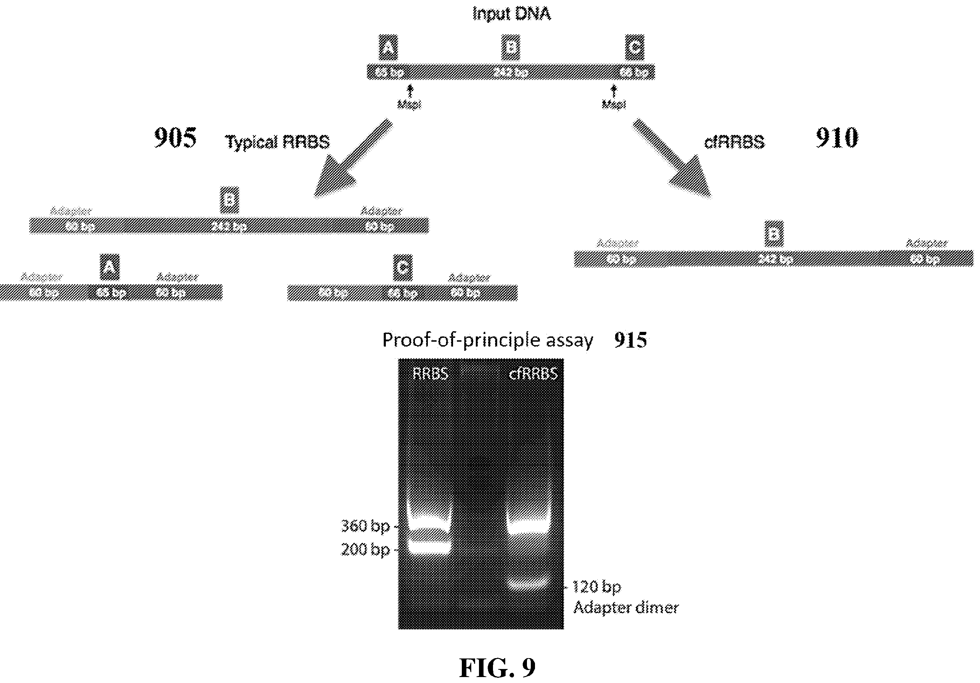

**FIG. 9** illustrates a comparison of gel electrophoresis of products from an RRBS assay and from a cfRRBS assay. Suppose an input DNA molecule comprises 3 different regions: an "A" region having a length of 65 bp, a "B" region having a length of 242 bp, and a "C" region having a length of 66 bp, with MspI restriction enzyme recognition sites ("cut sites") located at the boundaries between the "A" and "B" regions and between the "B" and "C" regions, and that the same input DNA molecule is processed using two different assays: a typical RRBS assay (in operation 905) and a cfRRBS assay (in operation 910) of the present disclosure.

In a typical RRBS assay (in operation 905), the MspI restriction enzyme digests the input DNA molecule at the two MspI restriction enzyme recognition sites, thereby fragmenting both region "A" and region "B" from the input DNA molecule to produce three separate "A," "B," and "C" fragments. Each of these three fragments is ligated to 60-bp adapters on both ends, thereby producing an adapter-ligated "A" fragment having a length of 185 bp, an adapter-ligated "B" fragment having a length of 362 bp, and an adapter-ligated "C" fragment having a length of 186 bp. This result may be undesirable for an input cfDNA molecule, since all three adapter-ligated fragments may be expected to be amplified efficiently, while only the "B" fragment contains the CpG-rich regions that are desired to be enriched.

In a cfRRBS assay (in operation 910) of the present disclosure, the input DNA molecule is first modified such that one end (the exposed ends) of each of the "A" region and the "C" region are rendered incapable of being coupled or ligated to adapters. Next, the MspI restriction enzyme digests the input DNA molecule at the two MspI restriction enzyme recognition sites, thereby fragmenting both region "A" and region "B" from the input DNA molecule to produce three separate "A," "B," and "C" fragments (as with the typical RRBS assay). However, in this case, only the "B" fragment is able to be adapter-ligated on both ends, while the "A" and "B" fragments are incapable of being adapter-ligated on both ends. Thus, the cfRRBS assay produces only an adapter-ligated "B" fragment having a length of 362 bp. This result may be desirable for an input cfDNA molecule, since only the "B" fragment, which contains the CpG-rich regions that are desired to be enriched, may be expected to be amplified efficiently.

In operation 915, gel electrophoresis is performed on products from both the typical RRBS assay and from the cfRRBS assay. As shown in FIG. 9, both the typical RRBS assay and the cfRRBS assay produce the desired product in the 360-bp range. However, the typical RRBS assay produces spurious products in the 200-bp range, while the cfRRBS assay produces no spurious products other than adapter dimers in the 120-bp range (which may be efficiently size selected to avoid unwanted subsequent amplification or other analysis). Hence, such a proof-of-principle assay demonstrates the advantages of performing a cfRRBS assay of the present disclosure to enrich CpG-rich regions cfDNA toward cfDNA methylation profiling.

### III. Methylation profiling of enriched DNA with CpG-rich regions

After a sample of cfDNA molecules has been enriched for CpG-rich regions, methylation profiling may be performed on the enriched DNA molecules. For example, sequencing reads may be generated from the enriched DNA molecules using any suitable sequencing method. The sequencing method can be a first-generation sequencing method, such as Maxam-Gilbert or Sanger sequencing, or a high-throughput sequencing (e.g., next-generation sequencing or NGS) method. A high-throughput sequencing method may sequence simultaneously (or substantially simultaneously) at least 10,000, 100,000, 1 million, 10 million, 100 million, 1 billion, or more polynucleotide molecules. Sequencing methods may include: pyrosequencing, sequencing-by-synthesis, single-molecule sequencing, nanopore sequencing, semiconductor sequencing, sequencing-by-ligation, sequencing-by-hybridization, Digital Gene Expression (Helicos), massively parallel sequencing, e.g., Helicos, Clonal Single Molecule Array (Solexa/Illumina), sequencing using PacBio, SOLiD, Ion Torrent, or Nanopore platforms, BGISEQ, or a combination thereof.

Described herein, the sequencing comprises whole genome sequencing (WGS). Described herein, the sequencing comprises whole genome bisulfite sequencing (WGBS), such as of reference DNA samples. Described herein, the sequencing comprises targeted sequencing using a panel containing a plurality of genetic loci. The sequencing may be performed at a depth sufficient to perform methylation profiling in a subject with a desired performance (e.g., accuracy, sensitivity, specificity, positive predictive value (PPV), negative predictive value (NPV), or the area under curve (AUC) of a receiver operator characteristic (ROC)). Described herein, the sequencing is performed at a depth of at least about 5X, at least about 10X, at least about 20X, at least about 50X, at least about 75X, at least about 100X, at least about 125X, at least about 150X, at least about 175X, or at least about 200X.

Described herein, the plurality of genetic loci may correspond to coding and/or non-coding genomic regions of a genome, such as CpG islands, hypermethylated regions and/or hypomethylated regions, and/or regions proximate to such hypermethylated regions and/or hypomethylated regions. The genomic regions may correspond to cancer-associated (or tumor-associated) coding and/or non-coding genomic regions of a genome, such as cancer driver mutations or genetic variants. Genetic variants may include, for example, single nucleotide variants (SNVs), copy number variants (CNVs), insertions or deletions (indels), fusion genes, hypermethylation, and hypomethylation.

Described herein, performing methylation profiling of a subject may comprise aligning the cfDNA sequencing reads to a reference genome. The reference genome may comprise at least a portion of a genome (e.g., the human genome). The reference genome may comprise an entire genome (e.g., the entire human genome). Described herein, the reference genome may comprise a plurality of genomic regions that correspond to coding and/or non-coding genomic regions of a genome, such as CpG-rich regions, CpG islands, hypermethylated regions and/or hypomethylated regions, and/or regions proximate to such hypermethylated regions and/or hypomethylated regions. The plurality of genomic regions may correspond to cancer-associated (or tumor-associated) coding and/or non-coding genomic regions of a genome, such as cancer driver mutations or genetic variants. Genetic variants may include, for example, single nucleotide variants (SNVs), copy number variants (CNVs), insertions or deletions (indels), fusion genes, hypermethylation, and hypomethylation. The alignment may be performed using, for example, a Burrows-Wheeler algorithm or other alignment algorithm (e.g., suitable for bisulfite converted reads).

Described herein, performing methylation profiling in a subject may comprise generating a quantitative measure of the cfDNA sequencing reads for each of a plurality of genetic loci. Quantitative measures of the cfDNA sequencing reads may be generated, such as counts of DNA sequencing reads that are aligned with a given genetic locus (e.g., a CpG-rich region, a CpG island, a hypermethylated region, a hypomethylated region, a region proximate to a hypermethylated regions, or a region proximate to a hypomethylated region). For example, cfDNA sequencing reads having a portion or all of the sequencing read aligning with a given CpG-rich region or CpG island may be counted toward the quantitative measure for that CpG-rich region or CpG island.

A combination of patterns of specific and non-specific CpG-rich regions and/or CpG islands may form a methylation profile of a subject. Changes over time in these patterns of CpG-rich regions and/or CpG islands may be indicative of changes in methylation profile of a subject. Such changes may comprise the presence of absence of methylation of one or more particular CpG sites, an increase in the level of methylation of a specific CpG-rich site or island, a decrease in the level of methylation of a specific CpG-rich site or island, and so forth.

Described herein, binding measurements may be performed for methylation profiling, which may comprise assaying enriched cfDNA fragments using probes that are selective for a plurality of CpG-rich regions and/or CpG islands in the plurality of enriched cfDNA fragments. Described herein, the probes are nucleic acid molecules having sequence complementarity with nucleic acid sequences of CpG-rich regions and/or CpG islands. Described herein, the nucleic acid molecules are primers or enrichment sequences. Described herein, the assaying comprises use of array hybridization or polymerase chain reaction (PCR), or nucleic acid sequencing.

Described herein, cfDNA molecules are enriched for at least a portion of the plurality of genetic loci. Described herein, the enrichment comprises amplifying the plurality of cfDNA molecules. For example, the plurality of cfDNA molecules may be amplified by selective amplification (e.g., by using a set of primers or probes comprising nucleic acid molecules having sequence complementarity with nucleic acid sequences of CpG islands). Alternatively or in combination, the plurality of cfDNA molecules may be amplified by universal amplification (e.g., by using universal primers). Described herein, the enrichment comprises selectively isolating at least a portion of the plurality of cfDNA molecules.

Described herein, performing methylation profiling in a subject comprises processing the sequence reads of enriched cfDNA fragments to obtain a quantitative measure of deviation. Described herein, the quantitative measure of deviation is a z-score relative to one or more reference cfDNA samples. The reference cfDNA samples may be obtained from subjects having a particular methylation profile and/or from subjects not having a particular methylation profile. The reference cfDNA samples may be obtained from subjects having a cancer type or from subjects not having a cancer type (e.g., pancreatic cancer, liver cancer, lung cancer, colorectal cancer, leukemia, bladder cancer, bone cancer, brain cancer, breast cancer, cervical cancer, endometrial cancer, esophageal cancer, gastric cancer, head and neck cancer, melanoma, ovarian cancer, testicular cancer, kidney cancer, sarcoma, bile duct cancer, thyroid cancer, gall bladder cancer, spleen cancer, and prostate cancer). The reference cfDNA samples may be obtained from subjects having a particular stage of a cancer or not having a particular stage of a cancer (including stage I, stage II, stage III, or stage IV). The reference cfDNA samples may be obtained from subjects having abnormal tissue-specific cell death.

Described herein, performing methylation profiling in a subject comprises determining a deviated cfDNA methylation profile of the subject when the quantitative measure of deviation satisfies a predetermined criterion. Described herein, the predetermined criterion is that a z-score (or a quantitative measure calculated from multiple z-scores) of the methylation profile of the subject is more or less than a predetermined number. The predetermined number may be about 0.1, about 0.2, about 0.5, about 1, about 1.5, about 2, about 2.5, about 3, about 3.5, about 4, about 4.5, about 5, or more than about 5.

Described herein, the plurality of genetic loci comprises CpG-rich regions, CpG islands, hypermethylated regions and/or hypomethylated regions, and/or regions proximate to such hypermethylated regions and/or hypomethylated regions. The plurality of genetic loci may comprise at least about 10 distinct genetic loci, at least about 20 distinct genetic loci, at least about 30 distinct genetic loci, at least about 40 distinct genetic loci, at least about 50 distinct genetic loci, at least about 75 distinct genetic loci, at least about 100 distinct genetic loci, at least about 500 distinct genetic loci, at least about 1 thousand distinct genetic loci, at least about 5 thousand distinct genetic loci, at least about 10 thousand distinct genetic loci, at least about 50 thousand distinct genetic loci, at least about 100 thousand distinct genetic loci, at least about 500 thousand distinct genetic loci, at least about 1 million distinct genetic loci, at least about 2 million distinct genetic loci, at least about 3 million distinct genetic loci, at least about 4 million distinct genetic loci, at least about 5 million distinct genetic loci, at least about 10 million distinct genetic loci, at least about 25 million distinct genetic loci, at least about 50 million distinct genetic loci, at least about 75 million distinct genetic loci, at least about 100 million distinct genetic loci, or more than 100 million distinct genetic loci. The location of the distinct genetic loci may or may not be in the same gene, on the same chromosome, or on different chromosomes.

Described herein, determining a deviated cfDNA methylation profile of the subject is performed with a sensitivity of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a deviated cfDNA methylation profile of the subject is performed with a specificity of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a deviated cfDNA methylation profile of the subject is performed with a positive predictive value (PPV) of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a deviated cfDNA methylation profile of the subject is performed with a negative predictive value (NPV) of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a deviated cfDNA methylation profile of the subject is performed with an area under curve (AUC) of a receiver operator characteristic (ROC) of at least about 0.5, at least about 0.6, at least about 0.7, at least about 0.75, at least about 0.8, at least about 0.85, at least about 0.9, at least about 0.95, at least about 0.96, at least about 0.97, at least about 0.98, or at least about 0.99.

Described herein, performing methylation profiling in a subject comprises determining a normal cfDNA methylation profile of the subject when the quantitative measure of deviation satisfies a predetermined criterion. Described herein, the predetermined criterion is that a z-score (or a quantitative measure calculated from multiple z-scores) of the methylation profile of the subject is more or less than a predetermined number. The predetermined number may be about 0.1, about 0.2, about 0.5, about 1, about 1.5, about 2, about 2.5, about 3, about 3.5, about 4, about 4.5, about 5, or more than about 5.

Described herein, determining a normal cfDNA methylation profile of the subject is performed with a sensitivity of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a normal cfDNA methylation profile of the subject is performed with a specificity of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a normal cfDNA methylation profile of the subject is performed with a positive predictive value (PPV) of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a normal cfDNA methylation profile of the subject is performed with a negative predictive value (NPV) of at least about 10%, at least about 20%, at least about 30%, at least about 40%, at least about 50%, at least about 60%, at least about 70%, at least about 80%, at least about 90%, at least about 95%, at least about 96%, at least about 97%, at least about 98%, or at least about 99%.

Described herein, determining a normal cfDNA methylation profile of the subject is performed with an area under curve (AUC) of a receiver operator characteristic (ROC) of at least about 0.5, at least about 0.6, at least about 0.7, at least about 0.75, at least about 0.8, at least about 0.85, at least about 0.9, at least about 0.95, at least about 0.96, at least about 0.97, at least about 0.98, or at least about 0.99.

Described herein, the subject has been diagnosed with cancer or is suspected of having cancer or is at risk for having cancer. For example, the cancer may be one or more types, including: brain cancer, breast cancer, cervical cancer, colorectal cancer, endometrial cancer, esophageal cancer, gastric cancer, hepatobiliary tract cancer, leukemia, liver cancer, lung cancer, lymphoma, ovarian cancer, pancreatic cancer, skin cancer, testicular cancer, kidney cancer, sarcoma, bile duct cancer, prostate cancer, thyroid cancer, gall bladder cancer, spleen cancer, or urinary tract cancer.

Described herein, based on the obtained cfDNA methylation profile of the subject (e.g., determining a deviated cfDNA methylation profile or a normal cfDNA methylation profile), methods of the present disclosure include administering a therapeutically effective dose of one or more treatments to treat the disease or disorder (e.g., cancer) of the subject. Described herein, the treatment comprises a chemotherapy, a radiation therapy, a targeted therapy, an immunotherapy, or a combination thereof. Based on the obtained methylation profile of the subject, an existing treatment of the subject may be discontinued and another treatment may be administered to the subject. Alternatively, based on the obtained methylation profile of the subject, an existing treatment of the subject may be continued and/or another treatment may be administered to the subject. An individual may be considered refractory to one or more treatments based on outcome of the methylation profile and as a result the treatment is never given or is given but is discontinued based on the outcome of subsequent methylation profiles for the same individual or is discontinued after a certain number of doses and/or period of time has passed.

An obtained cfDNA methylation profile of a subject may be assessed to determine a diagnosis of a cancer, prognosis of a cancer, or an indication of progression or regression of a tumor in the subject. In addition, one or more clinical outcomes may be assigned based on the cfDNA methylation profile assessment or monitoring (e.g., a difference in cfDNA methylation profile between two or more time points). Such clinical outcomes may include one or more of: diagnosing the subject with a cancer comprising tumors of one or more types, diagnosing the subject with the cancer comprising tumors of one or more types and/or stages, prognosing the subject with the cancer (e.g., indicating, prescribing, or administering a clinical course of treatment (e.g., surgery, chemotherapy, radiation therapy, hormone therapy, targeted therapy immunotherapy, or other treatment) for the subject, indicating, prescribing, or administering another clinical course of action (e.g., no treatment, continued monitoring such as on a prescribed time interval basis, stopping a current treatment, switching to another treatment) for the subject, or indicating an expected survival time for the subject.

Described herein, determining a cfDNA methylation profile for the subject comprises determining one or more predetermined thresholds for one or more genetic loci (e.g., a plurality of CpG-rich regions and/or CpG islands). The predetermined thresholds (e.g., for each of the plurality of CpG-rich regions and/or CpG islands) may be generated by performing the cfDNA methylation profiling on one or more samples from one or more control subjects (e.g., patients known to have or not have a certain disease or disorder, patients known to have or not have a certain tumor type, patients known to have or not have a certain tumor type of a certain stage, or healthy subjects not diagnosed with or exhibiting any clinical symptoms of a disease or disorder) and identifying a suitable predetermined threshold based on the cfDNA methylation profile of the control samples.

The predetermined thresholds may be adjusted based on a desired sensitivity, specificity, positive predictive value (PPV), negative predictive value (NPV), or accuracy of determining a deviated cfDNA methylation profile or determining a normal cfDNA methylation profile of a subject. For example, the predetermined threshold may be adjusted to be lower if a high sensitivity of determining a deviated cfDNA methylation profile status of a subject is desired. Alternatively, the predetermined threshold may be adjusted to be higher if a high specificity determining a deviated cfDNA methylation profile of a subject is desired. The predetermined threshold may be adjusted so as to maximize the area under curve (AUC) of a receiver operator characteristic (ROC) of the control samples obtained from the control subjects. The predetermined threshold may be adjusted so as to achieve a desired balance between false positives (FPs) and false negatives (FNs) in determining a deviated cfDNA methylation profile of subjects.
, determining a cfDNA methylation profile of a subject further comprises repeating the cfDNA methylation profiling at a second later time point. The second time point may be chosen for a suitable comparison of cfDNA methylation profile relative to the first time point. Examples of second time points may correspond to a time after surgical resection, a time during treatment administration or after treatment administration to treat the disease or disorder (e.g., cancer) in the subject to monitor efficiency of the treatment, or a time after the disease or disorder (e.g., cancer) is undetectable in the subject after treatment, e.g., to monitor for residual disease or cancer recurrence in the subject.

Described herein, determining a cfDNA methylation profile of a subject further comprises determining a difference between a first cfDNA methylation profile and a second cfDNA methylation profile, which difference is indicative of a progression or regression of a tumor of the subject. Alternatively, or in combination, the method may further comprise generating, by a computer processor, a plot of the first cfDNA methylation profile and the second cfDNA methylation profile as a function of the first time point and the second time point. The plot may be indicative of the progression or regression of the tumor of the subject. For example, the computer processor may generate a plot of the two or more cfDNA methylation profiles on a y-axis against the times corresponding to the time of collection for the data corresponding to the two or more cfDNA methylation profiles on an x-axis.

A determined difference or a plot illustrating a difference between the first cfDNA methylation profile and the second cfDNA methylation profile may be indicative of a progression or regression of a tumor of the subject. For example, if a deviation of the second cfDNA methylation profile is larger than that of the first cfDNA methylation profile, that difference may indicate, e.g., tumor progression, inefficacy of a treatment to the tumor in the subject, resistance of the tumor to an ongoing treatment, metastasis of the tumor to other sites in the subject, or residual disease or cancer recurrence in the subject. Alternatively, if a deviation of the second cfDNA methylation profile is smaller than that of the first cfDNA methylation profile, that difference may indicate, e.g., tumor regression, efficacy of a surgical resection of the tumor in the subject, efficacy of a treatment to the disease or disorder (e.g., cancer) in the subject, or lack of residual disease or cancer recurrence in the subject.

After assessing and/or monitoring cfDNA methylation profile, one or more clinical outcomes may be assigned based on the cfDNA methylation profile assessment or monitoring (e.g., a difference in cfDNA methylation profile between two or more time points). Such clinical outcomes may include one or more of: diagnosing the subject with a cancer comprising tumors of one or more types, diagnosing the subject with the cancer comprising tumors of one or more types and/or stages, prognosing the subject with the cancer (e.g., indicating, prescribing, or administering a clinical course of treatment (e.g., surgery, chemotherapy, radiation therapy, targeted therapy immunotherapy, or other treatment) for the subject, indicating, prescribing, or administering another clinical course of action (e.g., no treatment, continued monitoring such as on a prescribed time interval basis, stopping a current treatment, switching to another treatment) for the subject, or indicating an expected survival time for the subject.

### IV. Applications for Enriched DNA with CpG-rich regions

Described herein, a library generated using methods or systems encompassed herein to enrich for CpG-rich regions or CpG islands in cfDNA is utilized for an application. Described herein, the library is assayed for one or more characteristics. The library may be assayed to determine the amount and/or location of methylation site(s) in some or all of the molecules of the library. Described herein, the methylation pattern is determined for at least a portion in some or all of the molecules of the library, including one or more specific sites. Methylation profiling may be performed for at least a portion of some or all of the molecules of the library.

Described herein, the one or more methyation sites or markers may include plasma methylation biomarkers for various specific diseases or disorders, including cancers. The differentially methylated marker genes can be identified by comparing methylation profile data from patients with a certain disease or disorder characteristic (cancer type, stage, prognosis, treatment response, etc.) to methylation profile data from healthy controls. With a variety of methylation profiles specific to different cancers being identified, the disclosure herein relate to detecting many types of cancers and provide tumor location information for further specific clinical investigation based on a simple non-invasive liquid biopsy. Methylation profiles can be used to detect any disease or disorder based on a non-invasive liquid biopsy, for example.

Also described herein, cfDNA methylation profiles can be used to diagnose a subject or a patient based on determining whether the subject has a cfDNA methylation profile indicative of a disease or disorder. Described herein, there are methods of diagnosing a subject based on cfDNA methylation profile comprises generating a cfDNA methylation profile indicative of cancer whether the patient has cancer. Described herein, the cfDNA methylation profile is generated by processing a biological sample from the patient that comprises cell free DNA using methods, compositions and systems encompassed herein.

Described herein, cfDNA methylation profile(s) can be used to diagnose a patient who has symptoms of cancer, is asymptomatic of cancer, has a family or patient history of cancer, is at risk for cancer, or who has been diagnosed with cancer. Described herein, a patient may be a mammalian patient, such as a human. The cancer may be malignant, benign, metastatic, or a precancer. Described herein, the cancer is melanoma, non-small cell lung, small-cell lung, lung, hepatocarcinoma, retinoblastoma, astrocytoma, glioblastoma, gum, tongue, leukemia, neuroblastoma, head, neck, breast, pancreatic, prostate, renal, bone, testicular, ovarian, liver, mesothelioma, cervical, gastrointestinal, lymphoma, brain, colon, sarcoma, gall bladder thyroid, spleen, or bladder. The cancer may include a tumor comprised of tumor cells.

Described herein, there are methods for treating cancer in a cancer patient following determination of a need thereof based on methods and systems herein of enriching CpG island-comprising or CpG-rich DNA for cancer diagnosis. Such methods of treating may comprise administering to the patient an effective amount of chemotherapy, radiation therapy, hormone therapy, targeted therapy, or immunotherapy (or a combination thereof) after the patient has been determined to have cancer based on methods disclosed herein. The point of origin of the cancer may be determined, in which case, the treatment is tailored to cancer of that origin. Described herein, tumor resection is performed as the treatment or may be part of the treatment with one of the other treatments. Examples of chemotherapeutics include: alkylating agents such as bifunctional alkylators (for example, cyclophosphamide, mechlorethamine, chlorambucil, melphalan) or monofunctional alkylators (for example, dacarbazine (DTIC), nitrosoureas, temozolomide (oral dacarbazine)); anthracyclines (for example, daunorubicin, doxorubicin, epirubicin, idarubicin, mitoxantrone, and valrubicin; taxanes, which disrupt the cytoskeleton (for example, paclitaxel, docetaxel, abraxane, taxotere); epothilones; histone deacetylase inhibitors (for example, vorinostat, romidepsin); Topoisomerase I inhibitors (for example, irinotecan, topotecan); Topoisomerase II inhibitors (for example, etoposide, teniposide, tafluposide); kinase inhibitors (for example, bortezomib, erlotinib, gefitinib, imatinib, vemurafenib, and vismodegib); nucleotide analogs and nucleotide precursor analogs (for example, azacitidine. azathioprine, capecitabine, cytarabine, doxifluridine. fluorouracil, gemcitabine, hydroxyurea, mercaptopurine, methotrexate, tioguanine (formerly thioguanine); peptide antibiotics (for examples, bleomycin, actinomycin); platinum-based antineoplastics (for example, carboplatin, cisplatin, oxaliplatin); retinoids (for example, retinoin, alitretinoin, bexarotene); and, vinca alkaloids (for example, vinblastine, vincristine, vindesine, and vinorelbine). Examples of immunotherapies include, cellular therapy such as dendritic cell therapy (for example, involving chimeric antigen receptor); antibody therapy (for example, Alemtuzumab, Atezolizumab, Ipilimumab, Nivolumab, Ofatumumab, Pembrolizumab, Rituximab or other antibodies with the same target as one of these antibodies, such as CTLA-4, PD-1, PD-L1, or other checkpoint inhibitors ); and, cytokine therapy (for example, interferon or interleukin).

Described herein, methods of using cfDNA methylation profiling to diagnose a subject may further involve performing a biopsy, doing a CAT scan, doing a mammogram, performing ultrasound, or otherwise evaluating tissue suspected of being cancerous before or after determining the patient's methylation profile. Described herein, cancer that is found is classified in a cancer classification or staging (e.g., stage I, stage II, stage III, or stage IV).

Described herein, cfDNA methylation profiles obtained by methods and systems of enriching CpG islands in cfDNA is utilized for monitoring a therapy and/or monitoring tumor progression, including during and/or after treatment. For example, blood draws may be taken at various time points to monitor tumor progression throughout one or more treatment regimens, and the cfDNA therefrom may be assayed.

Described herein, cfDNA methylation profiles obtained by methods and systems of the present disclosure may be utilized for assessment of disease stage or as a prognostic biomarker, for example in cases where a tissue biopsy is not possible or where archived tumor samples are not available for genetic analysis.

Described herein, cfDNA methylation profiles obtained by methods and systems of enriching CpG-rich regions in cfDNA provided herein may be used for screening and early detection of cancer. For example, blood draws may be taken regularly from an individual without any symptoms of cancer to find cancer early or to ascertain a predisposition to cancer.

Described herein, cfDNA methylation profiles obtained by methods and systems of enriching CpG-rich regions in cfDNA provided herein may be used for prenatal testing of fetal DNA from maternal plasma or serum for identification of Down syndrome and other chromosomal abnormalities in a fetus.

Described herein, cfDNA methylation profiles obtained by methods and systems of enriching CpG-rich regions in cfDNA provided herein may be used for diagnosis of other type of diseases such as multiple sclerosis, traumatic/ischemic brain damage, diabetes, pancreatitis, or Alzheimer's disease.

The disclosure described herein can be implemented with respect to any method, system, kit, computer-readable medium, or apparatus as described herein, and *vice versa.* Furthermore, apparatuses described herein can be used to achieve methods described herein.

### V. Kits of the Disclosure

Any of the compositions described herein may be comprised in a kit. In a non-limiting example, cfDNA and/or one or more apparatuses for collection of cfDNA, enzymes, primers, ddNTPs, adapters, dNTPs, one or more blocking agents, bisulfite conversion reagents, buffers, other chemicals (including ATP, DTT, and so forth), or any combination thereof may be comprised in a kit.

The components of the kits may be packaged either in aqueous media or in lyophilized form. The kit may comprise a container, which may generally include at least one vial, test tube, flask, bottle, syringe, or other container into which a component may be placed, and in some cases, suitably aliquoted. Where there is a plurality of components in a kit, the kit also generally contains a second, a third, and/or other additional containers into which the additional components may be separately placed. However, various combinations of components may be comprised in a vial. The kits of the present disclosure may permit kit components to be contained in close confinement for commercial use. Such containers may include injection or blow-molded plastic containers into which the desired vials are retained.

Kits of the present disclosure may include instructions for performing methods described herein, such as methods for analyzing a plurality of cell-free deoxyribonucleic (DNA) molecules. Such instructions may be in physical form (e.g., printed instructions) or electronic form (e.g., web link for instructions for display on a user interface).

### VI. Examples

The following examples are presented in order to illustrate the disclosure.

### Example 1: Processing cell-free DNA using reduced representation bisulfite sequencing (cfRRBS)

Cancer cells can display aberrant DNA methylation patterns. Hypermethylated and/or hypomethylated tumor DNA fragments can be released into the bloodstream via processes such as cell apoptosis or necrosis, where they may become part of circulating cell-free DNA (cfDNA) in bodily fluids such as plasma or urine. Such cfDNA may be subjected to methylation profiling for clinical diagnostic applications such as cancer screening. For example, whole-genome bisulfite sequencing can provide a comprehensive view of the DNA methylome, but can be expensive to deep sequence the entire genome. While most fragments generated from genomic DNA and present in a typical RRBS library may have been cut twice by the restriction endonuclease (e.g., MspI), this may not hold true for cell-free DNA fragments. Therefore, performing typical RRBS on cell-free DNA may lead to challenges owing to limited CpG enrichment. Enriching cell-free DNA molecules for CpG-rich regions may advantageously allow methylation profiling toward clinical diagnostic applications.

The cfRRBS method may be illustrated by the following example workflow below.

First, 10 ng of input cell-free DNA (cfDNA) was extracted from plasma obtained from a subject. Next, the input cfDNA molecules were dephosphorylated and modified with dideoxynucleotides (ddNTP) moieties (labels). Next, the modified cfDNA molecules were then digested by 10 U of MspI restriction enzyme overnight to produce DNA fragments. Next, the DNA fragments were end repaired and dA tailed with 5 U of Klenow fragment exo⁻and a mixture of 1 millimolar (mM) dATP, 0.1 mM dGTP, and 0.1 mM dCTP. The dA-tailed DNA were then ligated with TruSeq multiplexing methylated adapters by T4 DNA ligase.

Barcodes (e.g., Unique Molecular Identifiers) can be added to facilitate the suppression of sequencing errors or PCR biases, in some cases. The ligation mixture, containing adapter-ligated DNA fragments, was purified with Agencourt AMPure XP beads and then subjected to bisulfite conversion. Next, the bisulfite-converted library was amplified for 20 cycles and size selected for a range of 150 to 400 bp. The prepared library contains highly enriched CpG-rich regions and CpG islands, which harbor essential methylation information, thereby significantly reducing the cfDNA sequencing cost and facilitates applications such as early diagnosis of cancer.

### Example 2: Preparation of reduced representation bisulfite sequencing libraries for cell-free DNA methylation profiling

As illustrated in FIG. 2, an example of a cfRRBS method may begin with dephosphorylating the input cfDNA molecules, such as by dephosphorylating 10 ng of input cfDNA with calf intestinal alkaline phosphatase (NEB), and then modifying the cfDNA molecules with a ddNTP moiety (for example, an "A," "C," "G," or "T," which may or may not be labeled), such as with 100 picomolar (pM) dideoxynucleotides (ddNTP) by 10 U terminal transferase (NEB).

Next, 10 U methylation insensitive restriction enzyme MspI (NEB) was used to digest the fragments at 37°C for 15 h. Next, 5 U of Klenow fragment exo- (NEB) and a mixture of 1 mM dATP, 0.1 mM dGTP, and 0.1 mM dCTP were used for end repair and dA-tailing by incubating with DNA at 30°C for 20 min, then 37 °C for 20 min. The dA-tailed DNA were then ligated with 500 nanomolar (nM) methylated stem-looped adapters by 30 Weiss U T4 DNA ligase (Thermoscientific^{®}) by incubating at 16°C for 20 h, and then incubated with USER^{™} enzyme (NEB) at 37°C for 15 min. Next, the ligation mixture, containing adapter-ligated DNA fragments, was purified using Agencourt AMPure XP beads (Beckman Coulter), and two rounds of bisulfite conversion were performed using an Epitect plus bisulfite kit (Qiagen). The libraries were amplified and indexed using a KAPA HiFi HotStart Uracil+ReadyMix PCR Kit for 12 cycles and then size selected for a range of 150-400 bp. The final library resembles TruSeq^{®} DNA and is compatible with Illumina platform sequencing.

By performing this procedure, DNA fragments containing zero or only one MspI recognizable sequence are discarded, while only fragments containing two or more MspI recognizable sequence are enriched. This procedure ensures that each enriched DNA fragment contains sequence reads with at least one CpG site, a result that allows cost-effective sequencing, which facilitates broad clinical application of diagnostic tools.

To test the performance of cfRRBS protocol, a study was conducted using a 373-bp input DNA fragment with a known sequence and containing two MspI digestion sites. A 100 ng input DNA sample was used to generate an RRBS library, while a 10 ng input DNA sample was used to generate a cfRRBS library. As shown in FIG. 9, unlike typical RRBS that enriches all 3 fragments (A, B, and C), cfRRBS is expected to only enrich fragment B, which has two MspI recognizable sequence on both ends.

The proof-of-concept of the cfRRBS workflow has been demonstrated by DNA gel electrophoresis of the prepared library. As shown in FIG. 9, the RRBS procedure generated both ~360 bp and ~260 bp fragments, while the cfRRBS procedure generated only ~360 bp fragments, as expected. There is a visible amount of ~120 bp adapter dimers formed in the cfRRBS library because of the low input DNA quantity. These adapter dimers may be efficiently removed, for example, by gel excision size selection of fragments having a length between 150 and 400 bp.

With reference to FIG. 3, in an example:
1. In operation 305, a 5' end of DNA is dephosphorylated, such as with calf intestinal alkaline phosphatase and modifies the 3' end of the DNA, such as with ddNTP. One purpose of this step is to impair the ability of the DNA molecule to be ligated to adapters to either end of the DNA molecule prior to MspI digestion. This is a useful step in cfRRBS library preparation to ensure that only fragments with MspI digestion sites on both ends can be ligated to adapters on both ends and hence amplified efficiently to generate the prepared library.
2. In operation 310, MspI digestion is performed on the adapter-ligated DNA molecules, generating DNA fragments that have an MspI site on both ends (center box), as well as fragments that have an MspI site on one end (left and right boxes).
3. In operation 310, the MspI-digested DNA fragments are prepared for adapter ligation. Since ddNTP-labeled ends are unable to be dA-tailed, those ends are incapable of being ligated to adapters in the next step. Therefore, the desired fragments can be dA-tailed on both ends, while other fragments (the fragments not desired to be amplified) can only be dA-tailed on one end or cannot be dA-tailed on both ends.
4. In operation 315, adapters (such as stem-looped adapters) are ligated to dA-tailed DNA fragments.
5. In operation 320, a USER^{™} (Uracil-Specific Excision Reagent; NEB) treatment is performed on the adapter-ligated DNA fragments in order to linearize the stem-loop by cutting the stem-loop into a linear shape, so that the two strands of DNA can be separated in the subsequent bisulfite conversion.
6. In operation 320, following linearization of stem-loops, bisulfite conversion is performed to convert unmethylated cytosine (C) residues to uracil residues (U).
7. In operation 325, only fragments with adapters on both ends (bottom box) can be amplified by PCR efficiently. The amplification of fragments with an adapter ligated only to one end is expected to be inefficient and negligible. Hence, the prepared cfRRBS library is expected to comprise only cfDNA fragments with MspI digestion sites on both ends, thereby producing DNA fragments enriched for CpG-rich regions. These DNA fragments are ready for methylation profiling (in operation 330).

### VII. Computer systems

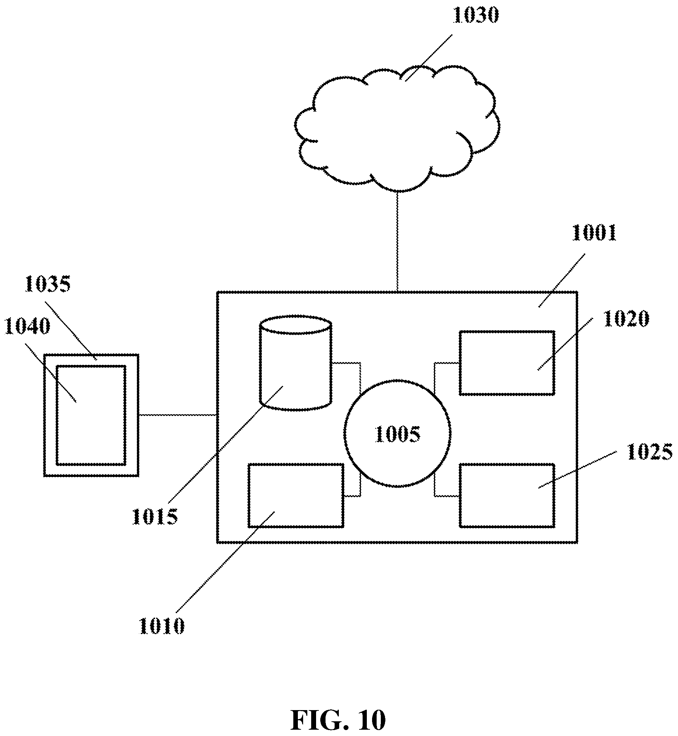

The present disclosure describes computer systems that are programmed to implement methods of the disclosure. **FIG. 10** shows a computer system 1001 that is programmed or otherwise configured to, for example, process sequence reads to (i) identify sequences from adapters at both ends of the sequence reads, and (ii) upon identifying the sequences, identify cell-free DNA molecules as having one or more CpG sites; measure a methylation status of DNA fragments to provide a methylation profile; process the methylation profile against a reference; and process a methylation profile to generate a likelihood of a subject as having or being suspected of having a disease or disorder. The computer system 1001 can regulate analysis, calculation, and/or generation as described in the present disclosure, such as, for example, processing sequence reads to (i) identify sequences from adapters at both ends of the sequence reads, and (ii) upon identifying the sequences, identify cell-free DNA molecules as having one or more CpG sites; measuring a methylation status of DNA fragments to provide a methylation profile; processing the methylation profile against a reference; and processing a methylation profile to generate a likelihood of a subject as having or being suspected of having a disease or disorder. The computer system 1001 can be an electronic device of a user or a computer system that is remotely located with respect to the electronic device. The electronic device can be a mobile electronic device.

The computer system 1001 includes a central processing unit (CPU, also "processor" and "computer processor" herein) 1005, which can be a single core or multi core processor, or a plurality of processors for parallel processing. The computer system 1001 also includes memory or memory location 1010 (e.g., random-access memory, read-only memory, flash memory), electronic storage unit 1015 (e.g., hard disk), communication interface 1020 (e.g., network adapter) for communicating with one or more other systems, and peripheral devices 1025, such as cache, other memory, data storage and/or electronic display adapters. The memory 1010, storage unit 1015, interface 1020 and peripheral devices 1025 are in communication with the CPU 1005 through a communication bus (solid lines), such as a motherboard. The storage unit 1015 can be a data storage unit (or data repository) for storing data. The computer system 1001 can be operatively coupled to a computer network ("network") 1030 with the aid of the communication interface 1020. The network 1030 can be the Internet, an intranet and/or extranet, or an intranet and/or extranet that is in communication with the Internet. The network 1030 in some cases is a telecommunication and/or data network. The network 1030 can include one or more computer servers, which can enable distributed computing, such as cloud computing. For example, one or more computer servers may enable cloud computing over the network 1030 ("the cloud") to perform analysis, calculation, and/or generation as described in the present disclosure, such as, for example, processing sequence reads to (i) identify sequences from adapters at both ends of the sequence reads, and (ii) upon identifying the sequences, identify cell-free DNA molecules as having one or more CpG sites; measuring a methylation status of DNA fragments to provide a methylation profile; processing the methylation profile against a reference; and processing a methylation profile to generate a likelihood of a subject as having or being suspected of having a disease or disorder. Such cloud computing may be provided by cloud computing platforms such as, for example, Amazon Web Services (AWS), Microsoft Azure, Google Cloud Platform, and IBM cloud. The network 1030, in some cases with the aid of the computer system 1001, can implement a peer-to-peer network, which may enable devices coupled to the computer system 1001 to behave as a client or a server.

The CPU 1005 can execute a sequence of machine-readable instructions, which can be embodied in a program or software. The instructions may be stored in a memory location, such as the memory 1010. The instructions can be directed to the CPU 1005, which can subsequently program or otherwise configure the CPU 1005 to implement methods of the present disclosure. Examples of operations performed by the CPU 1005 can include fetch, decode, execute, and writeback.

The CPU 1005 can be part of a circuit, such as an integrated circuit. One or more other components of the system 1001 can be included in the circuit. In some cases, the circuit is an application specific integrated circuit (ASIC).

The storage unit 1015 can store files, such as drivers, libraries and saved programs. The storage unit 1015 can store user data, e.g., user preferences and user programs. The computer system 1001 in some cases can include one or more additional data storage units that are external to the computer system 1001, such as located on a remote server that is in communication with the computer system 1001 through an intranet or the Internet.

The computer system 1001 can communicate with one or more remote computer systems through the network 1030. For instance, the computer system 1001 can communicate with a remote computer system of a user (e.g., a physician, a nurse, a caretaker, a patient, or a subject). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 1001 via the network 1030.

Methods as described herein can be implemented by way of machine (e.g., computer processor) executable code stored on an electronic storage location of the computer system 1001, such as, for example, on the memory 1010 or electronic storage unit 1015. The machine executable or machine readable code can be provided in the form of software. During use, the code can be executed by the processor 1005. In some cases, the code can be retrieved from the storage unit 1015 and stored on the memory 1010 for ready access by the processor 1005. In some situations, the electronic storage unit 1015 can be precluded, and machine-executable instructions are stored on memory 1010.

The code can be pre-compiled and configured for use with a machine having a processer adapted to execute the code, or can be compiled during runtime. The code can be supplied in a programming language that can be selected to enable the code to execute in a pre-compiled or as-compiled fashion.

The computer system 1001, can be embodied in programming. The technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 1001 can include or be in communication with an electronic display 1035 that comprises a user interface (UI) 1040 for providing, for example, a methylation profile, a report indicative of the methylation profile, and/or a likelihood of a subject as having or being suspected of having a disease or disorder. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit 1005. The algorithm can, for example, process sequence reads to (i) identify sequences from adapters at both ends of the sequence reads, and (ii) upon identifying the sequences, identify cell-free DNA molecules as having one or more CpG sites; measure a methylation status of DNA fragments to provide a methylation profile; process the methylation profile against a reference; and process a methylation profile to generate a likelihood of a subject as having or being suspected of having a disease or disorder.

## Claims

1. A method for enriching a plurality of deoxyribonucleic acid (DNA) fragments from a plurality of cell-free DNA (cfDNA) molecules of a subject, comprising:
(a) modifying one or both ends of each of at least a portion of said plurality of cell-free DNA molecules or derivatives thereof to provide a plurality of modified cell-free DNA molecules having ends that are incapable of coupling with adapters;
(b) subjecting said plurality of modified cell-free DNA molecules to restriction enzyme digestion to fragment each of at least a subset of said modified cell-free DNA molecules at one or more CpG sites, thereby producing a plurality of restriction enzyme-digested DNA fragments that contain one or more CpG sites and having ends that are capable of coupling with adapters; and
(c) coupling said adapters to ends of said plurality of restriction enzyme-digested DNA fragments to provide a plurality of tagged DNA fragments having methylated nucleic acid bases that are distinguishable from unmethylated nucleic acid bases,
wherein said modifying comprises:
(i) subjecting a 3' end of each of said at least said portion of said plurality of cfDNA molecules to conditions sufficient to modify said 3' end with a dideoxynucleotide (ddNTP) moiety or a functional analog thereof, and/or
(ii) incorporation of one or more blocker oligonucleotides at said one or both ends of each of at least a portion of said plurality of cfDNA molecules.

2. The method of claim 1, further comprising, prior to (c), separating fragments of said plurality of modified cfDNA molecules having said ends that are incapable of coupling with adapters from said plurality of restriction enzyme-digested DNA fragments, optionally wherein said fragments are coupled to magnetic beads, and wherein said fragments are separated using magnetic separation.

3. The method of claim 1, further comprising, after (c), separating fragments of said plurality of modified cfDNA molecules having said ends that are incapable of coupling with adapters from said plurality of tagged DNA fragments, optionally wherein said fragments are coupled to magnetic beads, and wherein said fragments are separated using magnetic separation.

4. The method of any one of claims 1-3, wherein in (a), ends of said plurality of modified cell-free DNA molecules are incapable of undergoing ligation or primer extension.

5. The method of any one of claims 1-4, further comprising, prior to (c), subjecting said plurality of restriction enzyme-digested DNA fragments to conditions sufficient to permit said methylated nucleic acid bases to be distinguished from said unmethylated nucleic acid bases, optionally wherein subjecting said plurality of restriction enzyme-digested DNA fragments to conditions sufficient to permit said methylated nucleic acid bases to be distinguished from said unmethylated nucleic acid bases comprises performing bisulfite conversion on said plurality of restriction enzyme-digested DNA fragments.

6. The method of any one of claims 1-4, further comprising, subsequent to (c), subjecting said plurality of tagged DNA fragments to conditions sufficient to permit said methylated nucleic acid bases to be distinguished from said unmethylated nucleic acid bases, thereby yielding an additional plurality of tagged DNA fragments, optionally wherein subjecting said plurality of tagged DNA fragments to said conditions sufficient to permit said methylated nucleic acid bases to be distinguished from said unmethylated nucleic acid bases comprises performing bisulfite conversion on said plurality of tagged DNA fragments.

7. The method of any one of claims 1-6, wherein said modifying comprises subjecting a 3' end of each of said at least said portion of said plurality of cfDNA molecules to conditions sufficient to modify said 3' end with a dideoxynucleotide (ddNTP) moiety or a functional analog thereof.

8. The method of any one of claims 1-7, wherein said modifying comprises incorporation of one or more blocker oligonucleotides at said one or both ends of each of at least a portion of said plurality of cfDNA molecules.

9. The method any one of claims 1-8, wherein said modifying further comprises subjecting a 5' end of each of said at least said portion of said plurality of cfDNA molecules to conditions sufficient to dephosphorylate said 5' end.

10. The method of any one of claims 1-9, wherein said restriction enzyme digestion is performed using one or more restriction enzymes that enrich for fragments having CpG sites, wherein said one or more restriction enzymes comprise MspI, HpaII, and/or TaqI.

11. The method of any one of claims 1-10, wherein each of said adapters comprises a functional sequence that is configured to couple to a flow cell of a nucleic acid sequencer.

12. The method of any one of claims 1-11, wherein coupling said adapters in (c) comprises ligating said adapters to said ends of said plurality of restriction enzyme-digested DNA fragments, optionally wherein the method further comprises, prior to said ligation, performing end repair or nucleic acid base tailing of said plurality of restriction enzyme-digested DNA fragments.

13. The method of claim 1, wherein said adapters are configured to be coupled to a nucleic acid molecule to provide a library for sequencing, optionally wherein said adapters are configured to be ligated to said nucleic acid molecule, optionally wherein said adapters comprise at least one stem-loop region, optionally wherein the method further comprises coupling said adapters to said nucleic acid molecule, and linearizing said stem-loop region of said adapters coupled to said nucleic acid molecule, optionally wherein said linearizing is performed using an endonuclease, a uracil glycosylase or a functional analog thereof, or a combination thereof, optionally wherein said endonuclease is endonuclease VIII or a functional analog thereof, optionally wherein said uracil glycosylase is a uracil deoxyribonucleic nucleic acid (DNA) glycosylase.

14. The method of any one of claims 1-13, wherein said adapters are Y shaped, are blunt ended, comprise a known sequence, or comprise a unique sequence that allows unique molecular identification of said plurality of tagged DNA fragments or derivatives thereof.

15. The method of any one of claims 1-14, further comprising performing size selection of said plurality of restriction enzyme-digested DNA fragments or said plurality of tagged DNA fragments to provide a size-selected plurality of DNA fragments, optionally wherein said size-selected plurality of DNA fragments have lengths from about 130 to about 400 nucleic acid bases or lengths from about 30 to about 250 nucleic acid bases, optionally wherein the method further comprises measuring a methylation status of at least a portion of said size-selected plurality of DNA fragments or said plurality of tagged DNA fragments, to provide a methylation profile of said at least said portion of said size-selected plurality of DNA fragments or said plurality of tagged DNA fragments.

## Patentansprüche

1. Verfahren zum Anreichern einer Mehrzahl von Desoxyribonukleinsäure(DNA)-Fragmenten aus einer Mehrzahl von zellfreien DNA(cIDNA)-Molekülen eines Subjekts, umfassend:
(a) Modifizieren eines oder beider Enden von jedem von mindestens einem Abschnitt der Mehrzahl von zellfreien DNA-Moleküle oder Derivaten davon, um eine Mehrzahl von modifizierten zellfreien DNA-Molekülen bereitzustellen, die Enden aufweisen, die nicht in der Lage sind, mit Adaptern zu koppeln;
(b) Unterziehen der Mehrzahl von modifizierten zellfreien DNA-Molekülen einem Restriktionsenzymverdau, um jedes von mindestens einer Untergruppe der modifizierten zellfreien DNA-Moleküle an einer oder mehreren CpG-Stellen zu fragmentieren, wodurch eine Mehrzahl von restriktionsenzymverdauten DNA-Fragmenten erzeugt wird, die eine oder mehrere CpG-Stellen enthalten und Enden aufweisen, die in der Lage sind, mit Adaptern zu koppeln; und
(c) Koppeln der Adapter an die Enden der Mehrzahl von restriktionsenzymverdauten DNA-Fragmenten, um eine Mehrzahl von markierten DNA-Fragmenten mit methylierten Nukleinsäurebasen bereitzustellen, die von unmethylierten Nukleinsäurebasen unterscheidbar sind,
wobei das Modifizieren umfasst:
(i) Unterziehen eines 3'-Endes von jedem des mindestens einen Abschnitts der Mehrzahl von cIDNA-Molekülen Bedingungen, die ausreichen, um das 3'-Ende mit einem Dideoxynukleotid(ddNTP)-Teil oder einem funktionellen Analogon davon zu modifizieren, und/oder
(ii) Einbau eines oder mehrerer Blocker-Oligonukleotide an das eine oder beide Enden von jedem von wenigstens einem Abschnitt der Mehrzahl von cIDNA-Molekülen.

2. Verfahren nach Anspruch 1, ferner umfassend vor (c) das Trennen von Fragmenten der Mehrzahl von modifizierten cIDNA-Moleküle mit den Enden, die nicht in der Lage sind, mit Adaptern zu koppeln, von der Mehrzahl von restriktionsenzymverdauten DNA-Fragmenten, wobei die Fragmente optional an magnetische Perlen gekoppelt sind, und wobei die Fragmente unter Verwendung einer magnetischen Trennung getrennt werden.

3. Verfahren nach Anspruch 1, ferner umfassend nach (c) das Trennen von Fragmenten der Mehrzahl von modifizierten cIDNA-Molekülen mit den Enden, die nicht in der Lage sind, mit Adaptern zu koppeln, von der Mehrzahl von markierten DNA-Fragmenten, wobei die Fragmente optional an magnetische Perlen gekoppelt sind, und wobei die Fragmente unter Verwendung einer magnetischen Trennung getrennt werden.

4. Verfahren nach einem der Ansprüche 1-3, wobei in (a) Enden der Mehrzahl von modifizierten zellfreien DNA-Molekülen nicht in der Lage sind, sich einer Ligation oder Primer-Verlängerung zu unterwerfen.

5. Verfahren nach einem der Ansprüche 1-4, ferner umfassend vor (c) das Unterwerfen der Mehrzahl von restriktionsenzymverdauten DNA-Fragmenten Bedingungen, die ausreichen, um zu erlauben, dass die methylierten Nukleinsäurebasen von den unmethylierten Nukleinsäurebasen unterschieden werden, wobei optional das Unterwerfen der Mehrzahl von restriktionsenzymverdauten DNA-Fragmenten Bedingungen, die ausreichen, um zu erlauben, dass die methylierten Nukleinsäurebasen von den unmethylierten Nukleinsäurebasen unterschieden werden, das Durchführen einer Bisulfit-Umwandlung an der Mehrzahl von restriktionsenzymverdauten DNA-Fragmenten umfasst.

6. Verfahren nach einem der Ansprüche 1-4, ferner umfassend, nach (c), das Unterwerfen der Mehrzahl von markierten DNA-Fragmenten Bedingungen, die ausreichen, um zu erlauben, dass die methylierten Nukleinsäurebasen von den unmethylierten Nukleinsäurebasen unterschieden werden, wodurch eine zusätzliche Mehrzahl von markierten DNA-Fragmenten erhalten wird, wobei optional das Unterwerfen der Mehrzahl von markierten DNA-Fragmente den Bedingungen, die ausreichen, um zu erlauben, dass die methylierten Nukleinsäurebasen von den unmethylierten Nukleinsäurebasen unterschieden werden, das Durchführen einer Bisulfit-Umwandlung an der Mehrzahl von markierten DNA-Fragmenten umfasst.

7. Verfahren nach einem der Ansprüche 1-6, wobei das Modifizieren das Unterwerfen eines 3'-Endes von jedem des mindestens einen Abschnitts der Mehrzahl von cIDNA-Molekülen Bedingungen umfasst, die ausreichen, um das 3'-Ende mit einem Dideoxynukleotid(ddNTP)-Teil oder einem funktionellen Analogon davon zu modifizieren.

8. Verfahren nach einem der Ansprüche 1-7, wobei das Modifizieren den Einbau von einem oder mehreren Blocker-Oligonukleotiden an einem oder beiden Enden von jedem von mindestens einem Abschnitt der Mehrzahl von cIDNA-Molekülen umfasst.

9. Verfahren nach einem der Ansprüche 1-8, wobei das Modifizieren ferner das Unterwerfen eines 5'-Endes jedes des mindestens einen Abschnitts der Mehrzahl von cIDNA-Molekülen Bedingungen umfasst, die ausreichen, um das 5'-Ende zu dephosphorylieren.

10. Verfahren nach einem der Ansprüche 1-9, wobei der Restriktionsenzymverdau unter Verwendung eines oder mehrerer Restriktionsenzyme durchgeführt wird, die für Fragmente mit CpG-Stellen anreichern, wobei das eine oder die mehreren Restriktionsenzyme MspI, HpaII und/oder TaqI umfassen.

11. Verfahren nach einem der Ansprüche 1-10, wobei jeder der Adapter eine funktionelle Sequenz umfasst, die dazu konfiguriert ist, an eine Durchflusszelle eines Nukleinsäuresequenzierers zu koppeln.

12. Verfahren nach einem der Ansprüche 1-11, wobei das Koppeln der Adapter in (c) das Ligieren der Adapter an die Enden der Mehrzahl von restriktionsenzymverdauten DNA-Fragmenten umfasst, optional wobei das Verfahren ferner vor der Ligation das Durchführen einer Endreparatur oder Nukleinsäurebasenschwanzbildung der Mehrzahl von restriktionsenzymverdauten DNA-Fragmenten umfasst.

13. Verfahren nach Anspruch 1, wobei die Adapter dazu konfiguriert sind, an eine Nukleinsäure gekoppelt zu werden, um eine Bibliothek für die Sequenzierung bereitzustellen, wobei die Adapter optional dazu konfiguriert sind, an das Nukleinsäuremolekül zu ligieren, wobei die Adapter optional mindestens eine einer Stamm-Schleifen-Region umfassen, wobei das Verfahren ferner das Koppeln der Adapter an das Nukleinsäuremolekül und das Linearisieren der Stamm-Schleifen-Region der an das Nukleinsäuremolekül gekoppelten Adapter umfasst, wobei das Linearisieren optional unter Verwendung einer Endonuklease, einer Uracil-Glycosylase oder eines funktionellen Analogons davon oder einer Kombination davon durchgeführt wird, wobei optional die Endonuklease Endonuklease VIII oder ein funktionelles Analogon davon ist, wobei optional die Uracil-Glycosylase eine Uracil-Desoxyribonukleinsäure(DNA)-Glycosylase ist.

14. Verfahren nach einem der Ansprüche 1-13, wobei die Adapter Y-förmig sind und ein stumpfes Ende haben, eine bekannte Sequenz umfassen oder eine einzigartige Sequenz umfassen, die eine einzigartige molekulare Identifizierung der Mehrzahl von markierten DNA-Fragmenten oder Derivaten davon ermöglicht.

15. Verfahren nach einem der Ansprüche 1-14, ferner umfassend das Durchführen einer Größenauswahl der Mehrzahl von restriktionsenzymverdauten DNA-Fragmenten oder der Mehrzahl von markierten DNA-Fragmenten, um eine größenausgewählte Mehrzahl von DNA-Fragmenten bereitzustellen, wobei optional die größenausgewählte Mehrzahl von DNA-Fragmenten Längen von etwa 130 bis etwa 400 Nukleinsäurebasen oder Längen von etwa 30 bis etwa 250 Nukleinsäurebasen aufweisen, wobei das Verfahren optional ferner das Messen eines Methylierungsstatus von mindestens einem Abschnitt der größenausgewählten Mehrzahl von DNA-Fragmenten oder der Mehrzahl von markierten DNA-Fragmenten, um ein Methylierungsprofil des wenigstens einen Abschnitts der größenausgewählten Mehrzahl von DNA-Fragmenten oder der Mehrzahl von markierten DNA-Fragmenten bereitzustellen.

## Revendications

1. Procédé pour enrichir une pluralité de fragments d'acide désoxyribonucléique (ADN) provenant d'une pluralité de molécules d'ADN acellulaire (ADNa) d'un sujet, comprenant les faits :
(a) de modifier une ou les deux extrémités de chacun d'au moins une partie de ladite pluralité de molécules d'ADN acellulaire ou dérivés de celles-ci pour fournir une pluralité de molécules d'ADN acellulaire modifiées ayant des extrémités qui sont incapables de se coupler à des adaptateurs ;
(b) de soumettre ladite pluralité de molécules d'ADN acellulaire modifiées à une digestion enzymatique de restriction pour fragmenter chacun d'au moins un sous-ensemble desdites molécules d'ADN acellulaire modifiées à un ou à plusieurs sites CpG, produisant ainsi une pluralité de fragments d'ADN ayant été soumis à une digestion enzymatique de restriction qui contiennent un ou plusieurs sites CpG et ayant des extrémités qui sont capables de se coupler à des adaptateurs ; et
(c) de coupler lesdits adaptateurs à des extrémités de ladite pluralité de fragments d'ADN ayant été soumis à une digestion enzymatique de restriction pour fournir une pluralité de fragments d'ADN étiquetés ayant des bases d'acide nucléique méthylées qui peuvent être distinguées de bases d'acide nucléique non méthylées,
dans lequel ledit fait de modifier comprend les faits :
(i) de soumettre une extrémité 3' de chacune de ladite au moins une partie de ladite pluralité de molécules d'ADNa à des conditions suffisantes pour modifier ladite extrémité 3' avec une fraction didésoxynucléotide (ddNTP) ou un analogue fonctionnel de celle-ci, et/ou
(ii) d'incorporer un ou plusieurs oligonucléotides bloqueurs à ladite une ou auxdites deux extrémités de chacun d'au moins une partie de ladite pluralité de molécules d'ADNa.

2. Procédé de la revendication 1, comprenant en outre, avant (c), le fait de séparer des fragments de ladite pluralité de molécules d'ADNa modifiées, ayant lesdites extrémités qui sont incapable de se coupler à des adaptateurs, de ladite pluralité de fragments d'ADN ayant été soumis à une digestion enzymatique de restriction, facultativement dans lequel lesdits fragments sont couplés à des billes magnétiques, et dans lequel lesdits fragments sont séparés en utilisant une séparation magnétique.

3. Procédé de la revendication 1, comprenant en outre, après (c), le fait de séparer des fragments de ladite pluralité de molécules d'ADNa modifiées, ayant lesdites extrémités qui sont incapables de se coupler à des adaptateurs, de ladite pluralité de fragments d'ADN étiquetés, facultativement dans lequel lesdits fragments sont couplés à des billes magnétiques, et dans lequel lesdits fragments sont séparés en utilisant une séparation magnétique.

4. Procédé de l'une quelconque des revendications 1 à 3, dans lequel dans (a), des extrémités de ladite pluralité de molécules d'ADN acellulaire modifiées sont incapables de subir une ligature ou une extension d'amorce.

5. Procédé de l'une quelconque des revendications 1 à 4, comprenant en outre, avant (c), le fait de soumettre ladite pluralité de fragments d'ADN ayant été soumis à une digestion enzymatique de restriction à des conditions suffisantes pour permettre auxdites bases d'acide nucléique méthylées d'être distinguées desdites bases d'acide nucléique non méthylées, facultativement dans lequel le fait de soumettre ladite pluralité de fragments d'ADN ayant été soumis à une digestion enzymatique de restriction à des conditions suffisantes pour permettre auxdites bases d'acide nucléique méthylées d'être distinguées desdites bases d'acide nucléique non méthylées comprend le fait de réaliser une conversion au bisulfite sur ladite pluralité de fragments d'ADN ayant été soumis à une digestion enzymatique de restriction.

6. Procédé de l'une quelconque des revendications 1 à 4, comprenant en outre, après (c), le fait de soumettre ladite pluralité de fragments d'ADN étiquetés à des conditions suffisantes pour permettre auxdites bases d'acide nucléique méthylées d'être distinguées desdites bases d'acide nucléique non méthylées, produisant ainsi une pluralité supplémentaire de fragments d'ADN étiquetés, facultativement dans lequel le fait de soumettre ladite pluralité de fragments d'ADN étiquetés auxdites conditions suffisantes pour permettre auxdites bases d'acide nucléique méthylées d'être distinguées desdites bases d'acide nucléique non méthylées comprend le fait de réaliser une conversion au bisulfite sur ladite pluralité de fragments d'ADN étiquetés.

7. Procédé de l'une quelconque des revendications 1 à 6, dans lequel ledit fait de modifier comprend le fait de soumettre une extrémité 3' de chacune de ladite au moins une partie de ladite pluralité de molécules d'ADNa à des conditions suffisantes pour modifier ladite extrémité 3' avec une fraction didésoxynucléotide (ddNTP) ou un analogue fonctionnel de celle-ci.

8. Procédé de l'une quelconque des revendications 1 à 7, dans lequel ledit fait de modifier comprend le fait d'incorporer un ou plusieurs oligonucléotides bloqueurs à ladite une ou auxdites deux extrémités de chacun d'au moins une partie de ladite pluralité de molécules d'ADNa.

9. Procédé l'une quelconque des revendications 1 à 8, dans lequel ledit fait de modifier comprend en outre le fait de soumettre une extrémité 5' de chacune de ladite au moins une partie de ladite pluralité de molécules d'ADNa à des conditions suffisantes pour déphosphoryler ladite extrémité 5'.

10. Procédé de l'une quelconque des revendications 1 à 9, dans lequel ladite digestion enzymatique de restriction est réalisée en utilisant un ou plusieurs enzymes de restriction qui permettent l'enrichissement en ce qui concerne des fragments ayant des sites CpG, dans lequel ledit un ou lesdits plusieurs enzymes de restriction comprennent MspI, HpaII, et/ou TaqI.

11. Procédé de l'une quelconque des revendications 1 à 10, dans lequel chacun desdits adaptateurs comprend une séquence fonctionnelle qui est configurée pour se coupler à une cellule de flux d'un séquenceur d'acide nucléique.

12. Procédé de l'une quelconque des revendications 1 à 11, dans lequel le fait de coupler lesdits adaptateurs dans (c) comprend le fait de ligaturer lesdits adaptateurs auxdites extrémités de ladite pluralité de fragments d'ADN ayant été soumis à une digestion enzymatique de restriction, facultativement dans lequel le procédé comprend en outre, avant ladite ligature, le fait de réaliser une réparation d'extrémité ou une extension homopolymérique de base d'acide nucléique de ladite pluralité de fragments d'ADN ayant été soumis à une digestion enzymatique de restriction.

13. Procédé de la revendication 1, dans lequel lesdits adaptateurs sont configurés pour être couplés à une molécule d'acide nucléique pour fournir une bibliothèque pour séquençage, facultativement dans lequel lesdits adaptateurs sont configurés pour être ligaturés à ladite molécule d'acide nucléique, facultativement dans lequel lesdits adaptateurs comprennent au moins une région tige-boucle, facultativement dans lequel le procédé comprend en outre les faits de coupler lesdits adaptateurs à ladite molécule d'acide nucléique, et de linéariser ladite région tige-boucle desdits adaptateurs couplés à ladite molécule d'acide nucléique, facultativement dans lequel le fait de linéariser est réalisé en utilisant une endonucléase, une uracile glycosylase, ou un analogue fonctionnel de celles-ci, ou une combinaison de celles-ci, facultativement dans lequel ladite endonucléase est l'endonucléase VIII ou un analogue fonctionnel de celle-ci, facultativement dans lequel ladite uracile glycosylase est une uracile acide nucléique désoxyribonucléique (ADN) glycosylase.

14. Procédé de l'une quelconque des revendications 1 à 13, dans lequel lesdits adaptateurs sont en forme de Y, sont à extrémités franches, comprennent une séquence connue, ou comprennent une séquence unique qui permet une identification moléculaire unique de ladite pluralité de fragments d'ADN étiquetés ou de dérivés de ceux-ci.

15. Procédé de l'une quelconque des revendications 1 à 14, comprenant en outre le fait de réaliser une sélection de taille de ladite pluralité de fragments d'ADN ayant été soumis à une digestion enzymatique de restriction ou de ladite pluralité de fragments d'ADN étiquetés pour fournir une pluralité ayant fait l'objet d'une sélection de taille de fragments d'ADN, facultativement dans lequel ladite pluralité ayant fait l'objet d'une sélection de taille de fragments d'ADN ont des longueurs d'environ 130 à environ 400 bases d'acides nucléique ou des longueurs d'environ 30 à environ 250 bases d'acides nucléique, facultativement dans lequel le procédé comprend en outre le fait de mesurer un état de méthylation d'au moins une partie de ladite pluralité ayant fait l'objet d'une sélection de taille de fragments d'ADN ou de ladite pluralité de fragments d'ADN étiquetés, pour fournir un profil de méthylation de ladite au moins une partie de ladite pluralité ayant fait l'objet d'une sélection de taille de fragments d'ADN ou de ladite pluralité de fragments d'ADN étiquetés.
